# EUROPEAN PATENT APPLICATION

(11) **EP 3 272 398 A1**
(43) Date of publication of application: **24.01.2018**
(21) Application number: 17188300.2
(22) Date of filing: 24.03.2011
(51) Int. Cl.: A61Q 19/00, A61K 8/11, A61K 8/64

(54) **LIPID NANOPARTICLE CAPSULES**

(30) Priority: 24.03.2010 ES 201030431
(62) Divisional of application: 11711041.1
(71) Applicant: Lipotec S.A., 08850 Gavà Barcelona (ES)
(72) Inventor: VILADOT PETIT, Josep LLuís, 08018 Barcelona (ES); DELGADO GONZÁLEZ, Raquel, 08850 Gavá (ES); FERNÁNDEZ BOTELLO, Alfonso, 29004 Málaga (ES)
(74) Representative: Carvajal y Urquijo, Isabel

(57) **Abstract**

Delivery system for active ingredients which comprises lipid nanoparticles, such as solid lipid nanoparticles (SLN) or nanostructured lipid carriers (NLC), polymerically coated, and their use in the preparation of pharmaceutical, cosmetic and/or alimentary compositions.

## Description

### FIELD OF THE INVENTION

This invention relates to a new delivery system for pharmaceutical, cosmetic and/or alimentary active ingredients which comprises lipid nanoparticles, such as solid lipid nanoparticles (SLN) or nanostructured lipid carriers (NLC), polymerically coated.

### BACKGROUND OF THE INVENTION

Solid lipid nanoparticles (SLN) constitute an alternative to other particulate systems for the delivery of active ingredients, such as emulsions, liposomes, micelles, microparticles and/or polymeric nanoparticles. SLN are generated by substituting the liquid lipid in the emulsions for a solid lipid, which means that the SLN are solid at room temperature as well as at body temperature.

The use of SLN as delivery systems enables the use of physiologically acceptable lipids, the possibility of avoiding the use of organic solvents in their preparation, and a wide range of routes of administration, which includes through the skin, orally or intravenously. As well as showing good bioavailability, their principal advantages are:
1. Protection of the active ingredient from chemical degradation. The lipid matrix of SLN can protect labile active ingredients from hydrolysis and/or oxidation, such as tocopherol, retinol and coenzyme Q10 [Gohla, S. et al. J. Microencapsul. 18: 149-158 (2001); Schäfer-Korting, M. et al. Adv. Drug Del. Rev. 59: 427-443 (2007)].
2. Based on the composition of the lipid particles, they offer control of the speed of active ingredient release and therefore the possibility of achieving sustained release profiles [Mehnert, W. et al. Eur. J. Pharm. Biopharm. 45: 149-155 (1998)].
3. Control of dehydration of the skin due to an occlusive effect [Müller, R.H. et al. J. Cosm. Sci. 52: 313-323 (2001)].
4. According to its components they can act as ultraviolet radiation filters [Müller, R.H. et al. Int. J. Cosm. Sci. 23: 233-243 (2001)].

A new generation of solid lipid nanoparticles are the nanostructured lipid carriers (NLC). These systems have the same advantages as the SLN, and also minimize or avoid some possible problems associated with SLN, such as the low loading capacity and active ingredient expulsion during storage. In contrast to the at least partially crystalline state of the lipid phase in SLN, NLC show a less organized solid lipid matrix. In the case of NLC, there are both solid and liquid compounds in the matrix, thus the greater disorganization leads to the existence of a greater number of holes with the subsequent increase in the ability to encapsulate active ingredients. For the preparation of NLC, sterically very different molecules of lipids are mixed together, mixtures of solid lipids with liquid lipids or oils [Müller, R. H. et al. Adv. Drug Deliv. Rev. 54 (Suppl. 1): S131-S155 (2002)].

The SLN and the NLC are colloidal systems which present the advantages of the liposomes and the microemulsions but are more effective for the protection of the active ingredient from chemical degradation and for its controlled release. They are 50 to 1000 nm in size and are kept stabilized in an aqueous suspension by hydrophilic surfactants and polymers. The principal characteristics of both, such as particle size, level of dispersion of the size, zeta potential, load efficiency and kinetic release, are determined by the nature of the lipid matrix, by the mixture of the surfactants, the viscosity of the lipid phase and the aqueous phase at the time of the emulsion preparation, and also by the general preparation conditions [Garzón, M.L. et al. Rev. Mex. Cien. Farm. 39(4): 50-66 (2008)].

The principal preparation mechanisms of these delivery systems, both SLN and NLC, are: high pressure homogenization (hot or cold), microemulsion with high speed stirring or ultrasound, emulsion through evaporation or diffusion of the solvent, double emulsion of water in oil in water (w/o/w) or emulsion through a contact membrane [Üner, M. et al. Int. J. Nanomed. 2: 289-300 (2007); Garzón, M.L. et al. Rev. Mex. Cien. Farm. 39(4): 50-66 (2008)].

The SLN have a solid lipid nucleus which can dissolve lipophilic drugs, which is the more common case for use. Examples of stabilized labile lipophilic cosmetic active ingredients in SLN would be coenzyme Q10 or retinol [Müller, R. H. et al. Adv. Drug Deliv. Rev. 54 (Suppl. 1): S131-S155 (2002)]. SLN can also be used with hydrophilic substances if they are combined with lipids forming conjugates: by formation of a salt (with a fatty acid) or by a covalent bond (forming ethers or esters with a fatty alcohol) [Garzón, M.L. et al. Rev. Mex. Cien. Farm. 39(1): 38-52 (2008)]. It is also possible to incorporate hydrophilic active ingredients in the lipid phase of NLC as an aqueous emulsion; this incorporation and the subsequent dispersion of the lipid in the external aqueous phase results in a multiple emulsion system of water in oil in water (w/o/w) [Müller, R.H. et al. WO 00/67728 A2].

The possibility of incorporating peptides in lipid particles could constitute a protection of the active ingredient from the proteolytic degradation in the gastrointestinal apparatus. However, there are few references of the use of lipids as matrix materials for formulations of peptides and proteins, due to the hydrophobic nature of the lipid matrix, which makes it more appropriate for incorporating lipophilic active ingredients than hydrophilic proteins. The use of emulsions to incorporate hydrophilic active ingredients such as insulin in SLN is described [Gallarate, M. et al. J. Microencapsul. 26: 394-402 (2009)]. In the publication of Gallarate *et al.* the preparation method of SLN implies the use of organic solvents, a factor which is problematic due to the possible retention of their residues. Gasco *et al.* incorporate thymopentin pentapeptide in solid lipid nanoparticles by two different methods: the formation of a lipophilic ion-pair with hexadecylphosphate, or by the formation of a multiple emulsion w/o/w dissolving the peptide in the internal aqueous phase [Gasco, M.R. et al. Int. J. Pharm. 132: 259-261 (1996)]; this latter method is also used by the same authors to incorporate a polypeptide derived from LHRH in SLN [Gasco, M.R. et al. Int. J. Pharm. 105: R1-R3 (1994)]. Zhou *et al.* describe an increase in the efficiency of encapsulation and the load capacity in the incorporation into SLN of different proteins using PLGA (lactic and glycolic acid copolymer) as an emulsifier [Zhou, W. et al. Colloids and Surfaces, B: Biointerfaces, 67: 199-204 (2008)].

The encapsulation of hydrophilic compounds in SLN or NLC presents another problem, as would be the diffusion of the active ingredient within the system towards a medium where it would be more soluble, i.e., towards the aqueous system in which the lipid nanoparticles are in suspension.

NLC and SLN are very suitable vehicles for the delivery of active ingredients through the skin. Better epidermal penetration of active ingredients is achieved when they are incorporated into SLN or NLC than when they are applied to the skin in the form of a solution or an emulsion. Thus, penetration in the stratum corneum is more effective when an aqueous dispersion of coenzyme Q10 incorporated into the SLN is applied than when solutions of the active ingredient in isopropanol or liquid paraffin are applied. In the same way, *in vitro* studies of epidermal penetration of NLC containing retinol in comparison with its nanoemulsions, revealed a penetration pattern different over time in both cases, the concentration of retinol in the skin being greater after 24 hours for the formulation which contained NLC [Müller, R. H. et al. Adv. Drug Deliv. Rev. 54 (Suppl. 1): S131-S155 (2002)].

Among the greatest problems of stability during storage of the SLN suspensions are the phenomena of gelification, aggregation and increase in the size of the particles and expulsion of the drug from the lipid matrix [Garzón, M.L. et al. Rev. Mex. Cien. Farm. 39(1): 38-52 (2008)]. On occasions, it is advisable to obtain a dry product by atomization or lyophilization.

Although the SLN and the NLC enable the chemical stability of the incorporated active ingredients to be improved, this stabilization is not complete. Surprisingly, the authors of this invention have found greater stabilization against degradation of cosmetic and/or pharmaceutical active ingredients incorporated into SLN or NLC when the SLN or NLC are polymerically coated.

Furthermore, even though epidermal penetration of the active ingredients incorporated into SLN or NLC is more effective than in solution or emulsion, the authors of this invention have found that epidermal penetration is still greater when the SLN or the NLC are polymerically coated, and also greater than for liposomes or micelles.

This invention proposes a delivery system based on lipid nanoparticles, such as solid lipid nanoparticles (SLN) or nanostructured lipid carriers (NLC), polymerically coated, which solves the problems presented by the classic systems described in the prior art. The delivery system of this invention avoids the diffusion of hydrophilic active ingredients in the SLN and NLC dispersions, enables greater stabilization of the incorporated active ingredient than in the SLN and the NLC, and has a greater epidermal penetration capacity than other known delivery systems.

### DESCRIPTION OF THE INVENTION

This invention provides a solution to the aforementioned problems. In a first aspect, this invention relates to a new delivery system which comprises lipid nanoparticles, selected from the group formed by solid lipid nanoparticles (SLN) or nanostructured lipid carriers (NLC), containing at least one active ingredient and which are polymerically coated.

The lipid nanoparticles can be found in the form of aqueous dispersion inside the delivery system. These lipid nanoparticles are constituted by a solid lipid matrix at room temperature, or by a matrix formed by a mixture of liquid (oils) and solid lipids at room temperature. The coating on the delivery system constitutes its external part and provides a complete and continuous coating of the lipid nanoparticles contained inside.

The delivery system of this invention contains active ingredients incorporated into its interior. The active ingredients incorporated into the delivery system of this invention can be, without restriction, cosmetic, pharmaceutical and/or alimentary active ingredients and/or adjuvants, among others.

The lipid nanoparticles in the delivery system contain the active ingredients incorporated into their lipid matrix. The active ingredients incorporated into lipid matrix can be lipophilic, hydrophilic or amphiphilic, and can be incorporated into the lipid matrix by solution or dispersion in the lipid, by adsorption on the surface of the lipid, or by dispersion of the active ingredient in the lipid in the form of an aqueous solution. The active ingredients can be solubilized in the lipid matrix by addition of surfactants, cyclodextrins or solvents which can optionally be totally or partially eliminated. When the active ingredient is hydrophilic, it can be incorporated into the system by prior formation of an emulsion or microemulsion by forming a w/o/w multiple emulsion system, or by forming a liposoluble ion pair.

Optionally, the delivery system can contain active ingredients incorporated into the external aqueous phase of the dispersion.

The polymeric coating of the delivery system of this invention constitutes an additional protection for the active ingredients, increasing their stability against chemical degradation by interaction with other components of the composition, by hydrolysis and/or oxidation due to the presence of oxygen and/or light. Furthermore, in the case of hydrophilic active ingredients such as peptides, the loss of the active ingredient by diffusion towards the external aqueous phase is avoided, as usually occurs in the aqueous dispersions of SLN or NLC. A greater percutaneous penetration of the active ingredients incorporated into the delivery system of the invention is also achieved with regards to microemulsions, liposomes, SLN or NLC.

The preparation processes of the delivery system of this invention consist of two stages: a) preparation of the lipid nanoparticles and b) encapsulation of the nanoparticles by polymeric coating, with both stages being able to be carried out in a single process.

The preparation processes of the lipid nanoparticles of the delivery system of this invention require, as a prior step, obtaining a molten mixture of the lipids by heating to a temperature higher than that of the melting point of the solid lipids. Next, the lipid nanoparticles can be formed by any of the methods described in the literature, preferably by those which do not involve the use of organic solvents, such as hot or cold high pressure homogenization [Müller, R.H. et al. EP 0605497 B2, WO 00/67728 A2, Eur. J. Pharm. Biopharm. 41: 62-69 (1995); Rehnert et al. Eur.J.Pharm.Biol. 45: 149-155 (1998)], or the microemulsion method [Gasco, M.R. et al. US 5250236 A].

For the preparation of lipid nanoparticles by means of the hot high pressure homogenization method, the mixture of molten lipids, their active ingredients or aqueous emulsions, and optionally emulsifying agents such as surfactants and cosurfactants, polymers and/or other excipients, are emulsified by stirring with a hot aqueous dissolution which can optionally contain other active ingredients, emulsifiers, polymers and/or other excipients. Subsequently, high pressure homogenization is carried out at a temperature higher than the melting points of the lipids. Finally the nanoemulsion obtained is cooled, obtaining the aqueous dispersion of lipid nanoparticles.

Using the cold homogenization method, the mixture of molten lipids, their active ingredients or aqueous emulsions, and optionally excipients, is cooled quickly by dry ice or liquid nitrogen. Thus the fragility of the lipid is increased to facilitate the subsequent grinding process, aimed at obtaining microparticles of 50-100 µm. These microparticles are dispersed in a cold aqueous solution which contains surfactants and which can optionally comprise other active ingredients, emulsifiers, polymers and/or other excipients. Finally, the dispersion obtained is subjected to high pressure homogenization at room temperature, or below it.

With the microemulsion method, the mixture of molten lipids, active ingredients, surfactants, cosurfactants and/or other excipients is microemulsified with hot water through stirring, and subsequently dispersed on a cold aqueous solution which can optionally contain other active ingredients, emulsifiers, polymers and/or other excipients, so the dispersion of lipid nanoparticles is formed.

The homogenization methods enable smaller lipid nanoparticles to be obtained and a lower amount of surfactants to be used.

In the case of hydrophilic active ingredients incorporated into a w/o/w multiple emulsion, the size of the internal nanoemulsion drops ranges between 0.1 and 100 nm, preferably between 1 and 50 nm, and more preferably between 10 and 20 nm.

In a particular embodiment, the size of the nanoparticles ranges between 1 and 1000 nm, preferably between 10 and 500 nm, and more preferably 100 to 200 nm.

In another particular embodiment, in the preparation process of the delivery system of this invention, the polymeric coating can be carried out by following the usual procedures in the prior art: physical-chemical procedures (simple coacervation, complex coacervation, simple or complex coacervation with pH change during reticulation, evaporation of the solvent), chemical procedures (interfacial polycondensation) and mechanical procedures (encapsulation in an air-fluidized bed). Preferably, the procedure used for the encapsulation of lipid nanoparticles of the delivery system of this invention is coacervation.

When encapsulation is carried out by coacervation, the procedure can be carried out in a single stage if a solution of the coacervation agent (simple coacervation) or another polymer (complex coacervation) is poured onto the dispersion of nanoparticles under stirring.

In another particular embodiment, in the formation of the polymeric coating of the delivery system of this invention a crosslinking agent is used. The crosslinking agent is selected, for example and not restricted to, from the group formed by aldehydes, glutaraldehyde, formaldehyde, transglutaminases, derivatives of methylenebisacrylamide, *N,N*-methylenebisacrylamide, *N,N*-(1,2-dihydroxyethylene)bisacrylamide, derivatives of ethylene glycol dimethacrylate, ethylene glycol diacrylate, diethylene glycol diacrylate, tetraethylene glycol diacrylate, ethylene glycol dimethacrylate, diethylene glycol dimethacrylate, triethylene glycol dimethacrylate, sodium tripolyphosphate, *N*-hydroxysuccinamide esters and/or imidoesters.

In another particular embodiment, the complex coacervation can be carried out with an increase in pH once the coacervate has been formed and before reticulation, which enables smaller capsules to be obtained.

The capsules of the delivery system of this invention can be recovered by the usual techniques, such as filtration, centrifugation, spray-drying and/or lyophilization.

In another particular embodiment, the size of the capsules of the delivery system of this invention ranges between 10 and 10000 nm, preferably between 50 and 5000 nm, and more preferably between 100-1000 nm.

The percentage of incorporation of active ingredient into the delivery system of this invention is quantitative.

In another particular embodiment, the liquid lipid of the delivery system of this invention has a melting point below 4 °C, and can be liquid or semi-liquid. The liquid lipid is selected, without restriction, from the group formed by vegetable oils, such as soybean oil, sunflower oil, corn oil, olive oil, palm oil, cottonseed oil, colza oil, peanut oil, coconut oil, castor oil, linseed oil, borage oil, evening primrose oil; marine oils, such as fish oils and algae oils; oils derived from petroleum, such as mineral oil, liquid paraffin and vaseline; short-chain fatty alcohols; medium-chain aliphatic branched fatty alcohols; fatty acid esters with short-chain alcohols, such as isopropyl myristate, isopropyl palmitate and isopropyl stearate and dibutyl adipate; medium-chain triglycerides (MCT) such as capric and caprylic triglycerides (INCI: Capric/caprylic triglycerides) and other oils in the Miglyol^{®} series; C₁₂-C₁₆ octanoates; fatty alcohol ethers, such as dioctyl ether, and/or mixtures thereof. Certain lipophilic active ingredients can also act as liquid lipid matrices at room temperature, for example and not restricted to, beta-carotene, vitamin E and retinol, and/or mixtures thereof.

In another particular embodiment, the solid lipid of the delivery system of this invention has a melting point above 37 °C. The solid lipid is selected, without restriction, from the group formed by solid triglycerides, such as trilaurin, tricaprylin, tripalmitin and tristearin, glyceryl trilaurate, glyceryl trimyristate or trimyristin, glyceryl tripalmitate, glyceryl tristearate, glyceryl behenate or tribehenin; solid diglycerides, such as dipalmitin and distearin; solid monoglycerides such as glyceryl monostearate; combinations of glycerides such as glyceryl palmitostearate, glyceryl stearate citrate and fats of the Witepsol^{®} series; long-chain aliphatic alcohols such as cetyl alcohol and stearic alcohol; medium and long-chain fatty acids (C₁₀-C₂₂) such as stearic acid, palmitic acid, behenic acid and capric acid, and their esters with polyols such as propylene glycol; fatty alcohol esters of long-chain fatty acids, such as cetyl palmitate, cetearyl olivate and hydroxyoctacosanyl hydroxystearate; sterols, cholesterol and cholesterol esters such as cholesteryl hemisuccinate, cholesteryl butyrate and cholesteryl palmitate; fatty amines such as stearyl amine; waxes such as beeswax, shea butter, cocoa butter, carnauba wax, ozokerite wax and paraffin wax; ceramides; hydrogenated vegetable oils such as hydrogenated castor oil, quaternary ammonium derivatives, such as behenyl trimethyl ammonium chloride (INCI: Alkyltrimethyl ammonium chloride), and/or mixtures thereof. Certain lipophilic active ingredients can also act as solid lipid matrices at room temperature, for example and not restricted to, Lipochroman-6 (INCI: Dimethylmethoxy chromanol), Chromabright (INCI: Dimethylmethoxy chromanyl palmitate), coenzyme Q10 and/or mixtures thereof.

The percentage of solid lipids is 100% in the SLN; in the mixtures of lipids of the NLC the liquid lipids and solid lipids are mixed in a proportion which ranges between 80:20 and 0.1:99.9, preferably between 50:50 and 0.1:99.9%, and even more preferably between 30:70 and 0.1:99.9.

The formation of micro- or nanoemulsions requires the addition of surfactants. In turn, the aqueous dispersions of lipid nanoparticles are stabilized by adding surfactants, cosurfactants, antiflocculants and/or viscosifiers, which favor the formation of nanoparticles at the same time as minimizing the formation of their aggregates.

In another particular embodiment, the surfactant is selected from the group formed by nonionic surfactants, amphoteric surfactants, anionic surfactants, cationic surfactants and/or mixtures thereof. The nonionic surfactant and/or amphoteric surfactant is selected, without restriction, from the group formed by lecithins, alkyl glycosides with an alkyl group that has from 6 to 24 carbon atoms, alkylmaltosides with an alkyl group that has from 6 to 24 carbon atoms, ethoxylated alkylphenols with an alkyl group that has from 6 to 24 carbon atoms and from 5 to 30 ethylene oxide units, alkylphenol polyoxyethylene ethers with an alkyl group that has from 6 to 24 carbon atoms, saturated and unsaturated fatty alcohols with an alkyl group that has from 8 to 24 carbon atoms, poloxamers, polysorbates, fatty acid esters with sugars, sorbitane esters, polyethylene glycol fatty acid esters, castor oil, fatty alcohol and polyoxyethylene ethers, fatty acid alkanolamides, amine oxides, alkyl betaines with an alkyl group that has from 6 to 24 carbon atoms, acyl amido betaines, alkylsulfobetaines with an alkyl group that has from 6 to 24 carbon atoms, glycine derivatives, digitonin, inulin lauryl carbamate and/or mixtures thereof. More preferably, the nonionic surfactant and/or amphoteric surfactant is selected from the group formed by octyl glucoside, decyl glucoside, lauryl glucoside, octyl fructoside, dodecyl maltoside, decyl maltoside, nonoxynol-9, polyethylene glycol *p*-(1,1,3,3-tetramethylbutyl)phenyl ether, palmityl alcohol, oleyl alcohol, poloxamer 188, poloxamer 407, polysorbate 20, polysorbate 60, polysorbate 80, methyl glucose dioleate, sorbitan monostearate or Span 60, sorbitan monolaurate or Span 20, sorbitan monopalmitate or Span 20, sorbitan olivate, polyethylene glycol 40 stearate, polyethylene glycol 50 stearate, polyethylene glycol 100 stearate, polyoxyethylene stearyl ether, polyoxyethylene lauryl ether, cocamide monoethanolamine, cocamide diethanolamine, cocamide triethanolamine, lauramide diethanolamine, lauramide monoethanolamine, cocamidopropylamine oxide, decyl betaine, dodecyl betaine, tetradecyl betaine, cocoyl betaine, cocamidopropyl betaine, cocamidopropyl hydroxysultaine, *N*-2-cocoyl amidoethyl hydroxyethylglycinate and *N*-2-cocoyl amidoethyl hydroxyethyl carboxy glycinate and/or mixtures thereof. The anionic surfactant is selected, without restriction, from the group formed by sulfonates such as alkylbenzene sulfonates, alkyl naphthalene sulfonates, ethoxylated fatty alcohol sulfonates, aliphatic sulfonates, hydroxy alkane sulfonates, alkyl glyceryl sulfonate ethers, perfluorooctane sulfonate; alkyl sulfosuccinates, alkyl sulfoacetates; alkyl sulfates such as sodium and ammonium lauryl sulfate, ethoxylated alkyl sulfates; fatty ester sulfates; ethoxylated fatty alcohol sulfates; alkyl ether sulfates; acyl isocyanates; pentafluorooctanoates; carboxylates; ethoxylated alkylphenols; ethanol ammonium salts; diethanolammonium, methylammonium, dimethylammonium, trimethylammonium; alkyl taurates, acyl or fatty acids; alkyl or acyl sarcosinates; phosphates such as phosphate esters, alkyl phosphates, polyoxyethylene lauryl ether phosphate; glutamates; stearates; biliary acids and their salts, such as glycocholic acid and sodium glycocholate, taurococholic acid and sodium taurocholate, taurodesoxycholate and/or mixtures thereof. The cationic surfactant is selected, without restriction, from the group formed by quaternary ammonium salts, such as cetyl trimethyl ammonium bromide, lauryl trimethyl ammonium chloride, benzyl dimethyl hexadecyl ammonium chloride, distearyl dimethyl ammonium chloride, dilauryl dimethyl ammonium chloride, dimyristyl dimethyl ammonium chloride, cetylpyridinium chloride, benzalkonium chloride, benzethonium chloride, methyl benzetonium chloride and/or mixtures thereof.

In another particular embodiment, the cosurfactant is selected, without restriction, from the group formed by low-molecular-weight alcohols and glycols, such as propanol, isopropanol, butanol and hexanol; long-chain fatty acids, such as octanoic acid and butyric acid; phosphoric acid monoesters; benzyl alcohol; biliary acid salts such as sodium cholate, sodium glycholate, sodium taurocholate, sodium taurodesoxycholate and/or mixtures thereof.

In another particular embodiment, the antiflocculant is selected, without restriction, from the group formed by sodium citrate, sodium pirophosphate, sodium sorbate, amphoteric surfactants, cationic surfactants and/or mixtures thereof.

In another particular embodiment, the viscosifier is selected, without restriction, from the group formed by cellulose ethers and esters, such as methyl cellulose, hydroxyethyl cellulose, hydroxypropyl cellulose and sodium carboxymethyl cellulose; polyvinyl derivatives, such as polyvinyl alcohol, polyvinylpyrrolidone and polyvinyl acetate; alginates; polyacrylates, xanthans; pectins and/or mixtures thereof.

In another particular embodiment, the polymer in the polymeric coating of the delivery system of this invention is selected, without restriction, from the group formed by proteins, polysaccharides, polyesters, polyacrylates, polycyanoacrylates, copolymers and/or mixtures thereof. Preferably, the polymeric coating of the microcapsules is selected from the group formed by gelatin, albumin, soy protein, pea protein, broad bean protein, potato protein, wheat protein, whey protein, β-lactoglobulin, caseinates, wheat starch, corn starch, zein, alginates, carrageenans, pectins, arabinogalactans, gum arabic, xanthan gum, mesquite gum, tragacanth gum, galactomannans, guar gum, carob seed gum, chitosan, agar, poly(L-lysine), dextran sulfate sodium, carboxymethyl galactomannan, carboxymethyl cellulose, methyl cellulose, ethyl cellulose, hydroxypropyl methyl cellulose (HPMC), cellulose nitrate, cellulose acetate butyrate, cellulose acetate phthalate, cellulose hydroxypropyl methyl phthalate, cellulose hydroxypropyl methyl acetate succinate, polyvinyl acetate phthalate, poly(ε-caprolactone), poly(*p*-dioxanone), poly(δ-valerolactone), poly(β-hydroxybutyrate), poly(β-hydroxybutyrate) and β-hydroxyvalerate copolymers, poly(β-hydroxypropionate), methylacrylic acid copolymers (Eudragit^{®} L and S), dimethylaminoethyl methacrylate copolymers (Eudragit^{®} E), trimethylammonium ethyl methacrylate copolymers (Eudragit^{®} RL and RS), lactic and glycolic acid polymers and copolymers, lactic and glycolic acid polymers and copolymers and polyethylene glycol and mixtures thereof.

Depending on the properties of the polymer used for the polymeric coating of the delivery system of this invention, it is possible to increase its specificity. A polymer that provides the polymeric coating with a positive charge enables the bond of the delivery system of this invention to the hair or textile materials to be increased. Optionally, the polymer in the coating of the delivery system of this invention can be a cationic polymer. The cationic polymer can be a natural or synthetic polymer, for example and not restricted to, cationic derivatives of cellulose, such as quaternized hydroxyethyl cellulose, which can be acquired under the name Polymer JR 400^{™} by Amerchol; cationic starches; diallyl ammonium and acrylamide salt copolymers; quaternized vinylpyrrolidone/vinylimidazole polymers such as Luviquat^{™} (BASF); condensation products of polyglycols and amines; polyquaternium polymers and copolymers; polymers called polyquaternium-6, polyquaternium-7, polyquaternium-16, polyquaternium-10 Merquats; polyquaternium-4 copolymers; dicocoylethylhydroxyethylammonium, grafting copolymers with a cellulose skeleton and quaternary ammonium groups; quaternized collagen polypeptides such as laurdimonium hydroxypropyl hydrolyzed collagen (Lamequat^{™} by Grünau); quaternized wheat polypeptides; polyethylenimine; cationic silicone polymers such as amidomethicone or quaternium-22 silicone; adipic acid and dimethylamino hydroxypropyl diethylenetriamine copolymers (Cartaretine^{™} by Sandoz); acrylic acid copolymers with dimethyldiallylammonium chloride (Merquat^{™} 550 by Chemviron); cationic chitin derivatives such as chitosan and its derivatives; condensation products of cationic dihalogen alkylene such as dibromobutane with bisdialkylamines; bis-dimethylamino-1,3-propane; derivatives of cationic guar gum such as guar-hydroxypropyltrimonium, Jaguar^{™} CBS, Jaguar^{™} C-17, Jaguar^{™} C-16 by Celanese; quaternary ammonium salt polymers such as Mirapol^{™}A-15, Mirapol^{™}AD-1, Mirapol^{™} AZ-1 by Miranol; quaternized polysaccharide polymers of natural derivatives such as azarose; cationic proteins selected from gelatin, gum arabic; cationic polymers from the group formed by polyamides, polycyanoacrylates, polylactides, polyglycolides, polyaniline, polypyrrole, polyvinylpyrrolidone, amino silicone polymers and copolymers, polystyrene, polyvinyl alcohol, polystyrene and maleic acid anhydride copolymers, methyl vinyl ether, epoxy resins and styrene and methyl methacrylate copolymers; dimethylamino methacrylate, cationic polyacrylates and polymethacrylates such as Eudragit^{™} RL 30 D by Röhm; polyamine derivatives optionally substituted by derivative polyethylene glycol members; polyamino acids under pH conditions wherein they are cationic; polyethyleneimine; quaternized derivatives of polyvinylpyrrolidone (PVP) and hydrophilic urethane polymers, as well as any mixture of the aforementioned cationic groups.

Optionally, the polymer in the coating of the delivery system of this invention can comprise a plasticizing additive. The plasticizing additive is selected, without restriction, from the group formed by citric acid alkyl esters such as triethyl citrate, tributyl citrate, acetyl tributyl citrate and acetyl triethyl citrate, phthalates such as butyl phthalate and diethyl phthalate, glycerin, sorbitol, maltitol, propylene glycol, polyethylene glycol, glucose, saccharose, lanolin, palmitic acid, oleic acid, stearic acid, fatty acid metal salts such as stearic acid or palmitic acid, sodium stearate, potassium stearate, propylene glycol monostearate, acetylated monoglycerides such as monoacetyl glycerin and glyceryl triacetate or triacetin, glyceryl lecithin, glyceryl monostearate, alkyl sebacates such as dibutyl sebacate or diethyl sebacate, alkyl fumarates, alkyl succinates, medium-chain triglycerides (MCT), castor oil, hydrogenated vegetable oils, waxes and/or mixtures thereof.

Optionally other technical additives of the polymer can be added which improve or facilitate the encapsulation process such as, for example, fluidizers, such as talc, colloidal silicon dioxide, glycerin, polyethylene glycol, glycerin monostearate and/or metal stearate salts.

The amount of active ingredient contained in the delivery system of this invention ranges between 0.00001 and 50% in weight, preferably between 0.0001 and 40% in weight, and more preferably between 0.001 and 30% in weight.

In another particular embodiment, the active ingredient in the delivery system of this invention is selected from the group formed by active ingredients and/or cosmetic and/or alimentary adjuvants. In particular, the active ingredients and/or cosmetic and/or alimentary adjuvants are selected, for example and not restricted to, from the group formed by surfactants, humectants or substances which retain moisture, moisturizers or emollients, agents stimulating healing, coadjuvant healing agents, agents stimulating re-epithelialization, coadjuvant re-epithelialization agents, agents which synthesize dermal or epidermal macromolecules, firming and/or redensifying and/or restructuring agents, cytokine growth factors, agents which act on capillary circulation and/or microcirculation, anti-glycation agents, free radical scavengers and/or anti-atmospheric pollution agents, reactive carbonyl species scavengers, 5α-reductase-inhibiting agents, lysyl- and/or prolyl hydroxylase inhibiting agents, defensin synthesis-stimulating agents, bactericidal agents and/or bacteriostatic agents and/or antimicrobial agents and/or germicidal agents and/or fungicidal agents and/or fungistatic agents and/or germ-inhibiting agents, anti-viral agents, antiparasitic agents, antihistaminic agents, NO-synthase inhibiting agents, desquamation agents or keratolytic agents and/or exfoliating agents, comedolytic agents, anti-psoriasis agents, anti-dandruff agents, anti-inflammatory agents and/or analgesics, anesthetic agents, anti-wrinkle and/or anti-aging agents, cosmetic and/or absorbent and/or body odor masking deodorants, antiperspirant agents, perfuming substances and/or perfumed oils and/or isolated aromatic compounds, anti-oxidizing agents, agents inhibiting vascular permeability, hydrolytic epidermal enzymes, whitening or skin depigmenting agents, agents inhibiting sweat-degrading enzymes, agents capable of filtering UV rays, agents which stimulate or regulate keratinocyte differentiation, anti-itching agents, agents which stimulate or inhibit the synthesis of melanin, propigmenting agents, self-tanning agents, agents stimulating the proliferation of melanocytes, liquid propellants, vitamins, amino acids, proteins, biopolymers, gelling polymers, skin relaxant agents, agents capable of reducing or treating bags under eyes, agents for the treatment and/or care of sensitive skin, astringent agents, agents regulating sebum production, anti-stretch mark agents, lipolytic agents or agents stimulating lipolysis, venotonic agents, anti-cellulite agents, calming agents, agents acting on cell metabolism, agents to improve dermal-epidermal junction, agents inducing hair growth or hair-loss retardants, body hair growth inhibiting or retardant agents, heat shock protein synthesis stimulating agents, muscle relaxants, muscle contraction inhibitory agents, agents inhibiting the aggregation of acetylcholine receptors, anticholinergic agents, elastase inhibitory agents, matrix metalloproteinase inhibitory agents, chelating agents, vegetable extracts, essential oils, marine extracts, mineral salts, cell extracts, emulsifying agents, agents stimulating the synthesis of lipids and components of the stratum corneum (ceramides, fatty acids, etc.), agents obtained from a bio-fermentation process and/or mixtures thereof. The nature of these active ingredients and/or cosmetic and/or alimentary adjuvants can be synthetic or natural, such as vegetable extracts, or come from a biotechnological process or from a combination of a synthetic process and a biotechnological process. Additional examples can be found in the CTFA International Cosmetic Ingredient Dictionary & Handbook, 12th Edition (2008*).* In the context of this invention, a biotechnological process is understood to be any process which produces the active ingredient, or part of it, in an organism, or in a part of it.

In a particular embodiment, the humectant or substance that retains moisture, moisturizer or emollient is selected, for example and not restricted to, from the group formed by polyols and polyethers such as glycerin, ethylhexylglycerin, caprylyl glycol, pentylene glycol, butylene glycol, propylene glycol and their derivatives, triethylene glycol, polyethylene glycol, Glycereth-26, Sorbeth-30; panthenol; pyroglutamic acid and its salts and derivatives; amino acids, such as serine, proline, alanine, glutamate or arginine; ectoine and its derivatives; *N*-(2-hydroxyethyl)acetamide; *N*-lauroyl-pyrrolidone carboxylic acid; *N*-lauroyl-L-lysine; *N*-alpha-benzoyl-L-arginine; urea; creatine; α- and β-hydroxy acids such as lactic acid, glycolic acid, malic acid, citric acid or salicylic acid, and their salts; polyglyceryl acrylate; sugars and polysaccharides, such as glucose, saccharide isomerate, sorbitol, pentaerythritol, inositol, xylitol, trehalose and derivatives thereof, sodium glucuronate, carraghenates (*Chondrus crispus*) or chitosan; glycosaminoglycans such as hyaluronic acid and derivatives thereof; aloe vera in any of its forms; honey; soluble collagen; lecithin and phosphatidylcholine; ceramides; cholesterol and its esters; tocopherol and its esters, such as tocopheryl acetate or tocopheryl linoleate; long-chain alcohols such as cetearyl alcohol, stearyl alcohol, cetyl alcohol, oleyl alcohol, isocetyl alcohol or octadecan-2-ol; long-chain alcohol esters such as lauryl lactate, myristyl lactate or C₁₂-C₁₅ alkyl benzoates; fatty acids such as stearic acid, isostearic acid or palmytic acid; polyunsaturated fatty acids (PUFAs); sorbitans such as sorbitan distearate; glycerides such as glyceryl monoricinoleate, glyceryl monostearate, glyceryl stearate citrate or caprylic and capric acid triglyceride; saccarose esters such as saccarose palmitate or saccarose oleate; butylene glycol esters, such as dicaprylate and dicaprate; fatty acid esters such as isopropyl isostearate, isobutyl palmitate, isocetyl stearate, isopropyl laurate, hexyl laurate, decyl oleate, cetyl palmitate, di-*n*-butyl sebacate, isopropyl myristate, isopropyl palmitate, isopropyl stearate, butyl stearate, butyl myristate, isopropyl linoleate, 2-ethylhexyl palmitate, 2-ethylhexyl cocoate, decyl oleate, myristyl myristate; squalene; mink oil; lanolin and its derivatives; acetylated lanolin alcohols; silicone derivatives such as cyclomethicone, dimethicone or dimethylpolysiloxane; Antarcticine^{®} [INCI: Pseudoalteromonas Ferment Extract] or acetyl-glutamyl-methionyl-alanyl-isoleucine, acetyl-arginyl-phenylglycyl-phenylglycine or acetyl-arginyl-6-aminohexanoyl-alanine marketed by Lipotec, petrolatum; mineral oil; mineral and synthetic waxes; beeswax (cera alba); paraffin; or waxes and oils with vegetable origins such as candelilla wax *(Euphorbia cerifera),* carnauba wax *(Copernicia cerifera),* shea butter *(Butirospermum parkii),* cocoa butter *(Theobroma cacao),* castor oil *(Ricinus communis),* sunflower oil *(Helianthus annuus),* olive oil *(Olea europaea),* coconut oil *(Cocos nucifera),* palm oil *(Elaeis guineensis),* wheat germ oil *(Triticum vulgare),* sweet almond oil *(Prunus amygdalus dulces),* musk rose oil *(Rosa moschata),* soya bean oil *(Glycine soja),* grape seed oil *(Vitis vinifera),* calendula oil *(Calendula officinalis),* jojoba oil *(Simmonsis chinensis),* mango oil *(Mangifera indica),* avocado oil *(Persea gratissima),* and/or mixtures thereof, among others.

Likewise, in another particular embodiment, the agent stimulating healing, coadjuvant healing agent, agent stimulating re-epithelialization and/or coadjuvant re-epithelialization agent is selected, for example and not restricted to, from the group formed by extracts of *Aristoloquia clematis, Centella asiatica, Rosa moschata, Echinacea angustifolia, Symphytum officinale, Equisetum arvense, Hypericum perforatum, Mimosa tenuiflora, Persea gratisima, Prunus africanum, Tormentilla erectea, Aloe vera,* Polyplant^{®} Epithelizing [INCI: Calendula Officinalis, Hypericum Perforatum, Chamomilla Recutita, Rosmarinus Officinalis] marketed by Provital, Cytokinol^{®} LS 9028 [INCI: Hydrolyzed Casein, Hydrolyzed Yeast Protein, Lysine HCl] marketed by Laboratories Serobiologiques/Cognis or Deliner^{®} [INCI: Zea May (Corn) Kernel Extract] marketed by Coletica/Engelhard/BASF, allantoin, cadherins, integrins, selectins, hyaluronic acid receptors, immunoglobulins, fibroblast growth factor, connective tissue growth factor, platelet-derived growth factor, vascular endothelial growth factor, epidermal growth factor, insulin-like growth factor, keratinocyte growth factors, colony-stimulating factors, transforming growth factor beta, tumor necrosis factor alpha, interferons, interleukins, matrix metalloproteinases, receptor protein tyrosine phosphatases, Antarcticine^{®} [INCI: Pseudoalteromonas Ferment Extract], Decorinyl^{®} [INCI: Tripeptide-10 Citrulline], Trylagen^{®} [INCI: Pseudoalteromonas Ferment Extract, Hydrolyzed Wheat Protein, Hydrolyzed Soy Protein, Tripeptide-10 Citrulline, Tripeptide-1], acetyl-glutamyl-methionyl-alanyl-isoleucine, acetyl-arginyl-phenylglycyl-phenylglycine or acetyl-arginyl-6-aminohexanoyl-alanine marketed by Lipotec, among others.

In a particular embodiment, the agent stimulating dermal or epidermal macromolecular synthesis is selected, for example and not restricted to, from the group formed by agents stimulating collagen synthesis, agents stimulating elastin synthesis, agents stimulating decorin synthesis, agents stimulating laminin synthesis, agents stimulating chaperone synthesis, agents stimulating hyaluronic acid synthesis, agents stimulating aquaporin synthesis, agents stimulating fibronectin synthesis, agents inhibiting collagen degradation, agents inhibiting elastin degradation, agents inhibiting serine proteases such as leukocyte elastase or cathepsin G, agents stimulating fibroblast proliferation, agents stimulating adipocyte proliferation, agents stimulating adipocyte differentiation, agents stimulating angiogenesis, agents stimulating glycosaminoglycan synthesis, DNA repair agents and/or DNA protecting agents, for example and not restricted to, extracts of *Centella asiatica, Saccharomyces cerevisiae, Solanum tuberosum, Rosmarinus officinalis, Vaccinium angustifolium,* extract of the algae *Macrocystis pyrifera, Padina pavonica,* extract of the plants soy, malt, flax, sage, red clover, kakkon-to, white lupin, hazelnut extract, corn extract, yeast extract, extract of beech tree shoots, extract of leguminosae seeds, extract of plant hormones such as gibberellins, auxins or cytokinins among others, or extract of zooplankton Salina, the product of milk fermentation with *Lactobacillus Bulgaricus,* asiaticosides and derivatives thereof, vitamin C and derivatives thereof, cinnamic acid and derivatives thereof, Matrixyl^{®} [INCI: Palmitoyl Pentapeptide-3], Matrixyl^{®} 3000 [INCI: Palmitoyl Tetrapeptide-3, Palmitoyl Oligopeptide] or Biopeptide CL^{™} [INCI: Glyceryl Polymethacrylate, Propylene Glycol, Palmitoyl Oligopeptide] marketed by Sederma, Antarcticine^{®} [INCI: Pseudomonas Ferment Extract], Decorinyl^{®} [INCI: Tripeptide-10 Citrulline], Serilesine^{®} [INCI: Hexapeptide-10], Lipeptide [INCI: Hydrolized vegetable protein], Aldenine^{®} [INCI: Hydrolized Wheat Protein, Hydrolized Soy Protein, Tripeptide-1], Peptide AC29 [INCI: Acetyl Tripeptide-30 Citrulline], acetyl-arginyl-phenylglycyl-tryptophyl-phenylglycine, acetyl-arginyl-phenylglycyl-valyl-glycine or acetyl-arginyl-phenylglycyl-valyl-phenylglycine marketed by Lipotec, Drieline^{®} PF [INCI:Yeast Betaglucan] marketed by Alban Muller, Phytovityl C^{®} [INCI: Aqua, Zea Mays Extract] marketed by Solabia, Collalift^{®} [INCI: Hydrolyzed Malt Extract] marketed by Coletica/Engelhard, Phytocohesine^{®} PSP [proposed INCI: Sodium Beta-Sitosterol Sulfate] marketed by Seporga, minerals such as calcium among others, retinoids and derivatives thereof, isoflavonoids, carotenoids, in particular lycopene, pseudodipeptides, retinoids and derivatives thereof such as retinol or retinyl palmitate among others, or heparinoids among others.

In a particular embodiment, the elastase-inhibiting agent is selected, for example and not restricted to, from the group formed by Elhibin^{®} [INCI: Glycine Soja (Soybean) Protein], Preregen^{®} [INCI: Glycine Soja (soybean) Protein, Oxido Reductases] or Regu^{®}-Age [INCI:Hydrolyzed Rice Bran Protein, Glycine Soja (Soybean) Protein, Oxido Reductases] marketed by Pentapharm/DSM, Juvenesce [INCI: Ethoxydiglicol and caprylic Triglycerid, Retinol, Ursolic Acid, Phytonadione, Ilomastat], Micromerol^{™} [INCI: Pyrus Malus Extract], Heather Extract [INCI: Calluna Vulgaris Extract], Extracellium^{®} [INCI: Hydrolyzed Potato Protein] or Flavagrum^{™} PEG [INCI: PEG-6 Isostearate, Hesperetin Laurate] marketed by Coletica/Engelhard/BASF, Proteasyl^{®} TP LS8657 [INCI: Pisum Sativum Extract] marketed by Laboratoires Sérobiologiques/Cognis, acetyl-arginyl-phenylglycyl-tryptophyl-phenylglycine, acetyl-arginyl-phenylglycyl-valyl-glycine or acetyl-arginyl-phenylglycyl-valyl-phenylglycine marketed by Lipotec, Sepilift DPHP [INCI: Dipalmitoyl hydroxyproline] marketed by SEPPIC, Vitaderm^{®} [INCI: Alcohol, Water, Glycerin, Hydrolyzed Rice Protein, Ilex Aquifolium Extract, Sodium Ursolate, Sodium Oleanolate] marketed by Rahn, Gatuline^{®} Age Defense 2 [INCI: Juglans Regia (Walnut) Seed Extract] marketed by Gattefosse, IP 2000 [INCI: Dextran, Trifluoroacetyl Tripeptide-2] marketed by IEB and Atrium, Radicaptol [INCI: Propylene Glycol, Water, Passiflora Incarnata Flower Extract, Ribes Nigrum (Blackcurrant) Leaf Extract, Vitis Vinifera (grape) Leaf Extract] marketed by Solabia or ViaPure^{™} Boswellia [INCI: Olivanum (Boswellia Serrata) Extract] marketed by Soliance, among others.

In a particular embodiment, the matrix metalloproteinase-inhibiting agent is selected, for example and not restricted to, from the group formed by ursolic acid, isoflavones such as genistein, quercetin, carotenoids, lycopene, soy extract, cranberry extract, rosemary extract, *Trifolium pratense* (red clover) extract, *Phormium tenax* (New Zealand flax) extract, kakkon-to extract, sage extract, retinol and derivatives thereof, retinoic acid and derivatives thereof, sapogenins such as diosgenin, hecogenin, smilagenin, sarsapogenin, tigogenin, yamogenin and yucagenin among others, Collalift^{®} [INCI: Hydrolyzed Malt Extract], Juvenesce [INCI: Ethoxydiglicol and Caprylic Triglyceride, Retinol, Ursolic Acid, Phytonadione, Ilomastat] or EquiStat [INCI Pyrus Malus Fruit Extract, Glycine Soja Seed Extract] marketed by Coletica/Engelhard, Pepha^{®}-Timp [INCI: Human Oligopeptide-20], Regu-Age [INCI: Hydrolyzed Rice Bran Protein, Glycine Soja Protein, Oxido Reductases] or Colhibin [INCI: Hydrolyzed Rice Protein] marketed by Pentapharm, Lipeptide [INCI: Hydrolized vegetable protein] or Peptide AC29 [INCI: Acetyl Tripeptide-30 Citrulline] marketed by Lipotec, Litchiderm^{™} [INCI: Litchi Chinensis pericarp extract] or Arganyl^{™} [INCI: Argania Spinosa Leaf Extract] marketed by Laboratories Sérobiologiques/Cognis, MDI Complex^{®} [INCI: glycosaminoglycans] or ECM-Protect^{®} [INCI: Water (Aqua), Dextran, Tripeptide-2] marketed by Atrium Innovations, Dakaline [INCI: Prunus amygdalus dulcis, Anogeissus leiocarpus bark extract] marketed by Soliance, Homeostatine [INCI: Enteromorpha compressa, Caesalpinia Spinosa] marketed by Provital, Timp-Peptide [proposed INCI: Acetyl Hexapeptide] or ECM Moduline [proposed INCI: Palmitoyltripeptide] marketed by Infinitec Activos, IP2000 [INCI: Dextran, Trifluoroacetyl tripeptide-2] marketed by Institut Europeen de Biologie Cellulaire, Actimp 1.9.3^{®} [INCI: Hydrolyzed Lupine Protein] marketed by Expanscience Laboratories, Vitaderm^{®} [INCI: Alcohol, Water (Aqua), Glycerin, Hydrolyzed Rice Protein, Ilex Aquifolium Extract, Sodium Ursolate, Sodium Oleanolate] marketed by Rahn, adapalene, tetracyclines and derivatives thereof such as minocycline, rolitetracycline, chlortetracycline, metacycline, oxytetracycline, doxycycline, demeclocycline and their salts, Batimastat [BB94; [4-(*N*-hydroxyamino) -2*R*-isobutyl-3*S*-(thiophene-2-ylthiomethyl)succinyl]-L-phenylalanine-*N*-methylamide], Marimastat [BB2516; [2*S*-[*N*4(*R**),2*R**,3*S*]]-*N*4[2,2-dimethyl-1-[methylaminocarbonyl]propyl]-*N*1,2-dihydroxy-3-(2-methylpropyl)butanediamide], among others.

In a particular embodiment, the firming and/or redensifying and/or restructuring agent is selected, for example and not restricted to, from the group formed by extracts of *Malpighia punicitolia, Cynara scolymus, Gossypium herbaceum, Aloe Barbadensis, Panicum miliaceum, Morus nigra, Sesamum indicum, Glycine soja, Triticum vulgare,* Pronalen^{®} Refirming HSC [INCI: Triticum vulgare, Silybum Marianum, Glycine Soy, Equisetum Arvense, Alchemilla Vulgaris, Medicago Sativa, Raphanus Sativus] or Polyplant^{®} Refirming [INCI: Coneflower, Asiatic Centella, Fucus, Fenugreek] marketed by Provital, Lanablue^{®} [INCI: Sorbitol, Algae Extract] marketed by Atrium Innovations, Pepha^{®}-Nutrix [INCI Natural Nutrition Factor] marketed by Pentapharm, or vegetable extracts which contain isoflavones, Biopeptide EL^{™} [INCI: Palmitoyl Oligopeptide], Biopeptide CL^{™} [INCI: Palmitoyl Oligopeptide], Vexel^{®} [INCI: Water (Aqua), Propylene Glycol, Lecithin, Caffeine, Palmitoyl Carnitine], Matrixyl^{®} [INCI: Palmitoyl Pentapeptide-3], Matrixyl^{®} 3000 [INCI: Palmitoyl Tetrapeptide-3, Palmitoyl Oligopeptide] or Bio-Bustyl^{™} [INCI: Glyceryl Polymethacrylate, Rahnella Soy Protein Ferment, Water (Aqua), Propylene Glycol, Glycerin, PEG-8, Palmitoyl Oligopeptide] marketed by Sederma, Dermosaccharides^{®} HC [INCI: Glycerin, Water (Aqua), Glycosaminoglycans, Glycogen], Aglycal^{®} [INCI: Mannitol, Cyclodextrin, Glycogen, Aratostaphylos Uva Ursi Leaf Extract], Cytokinol^{®} LS [INCI: Hydrolyzed Casein, Hydrolyzed Yeast Protein, Lysine HCL] or Firmiderm^{®} LS9120 [INCI: Terminalia Catappa Leaf extract, Sambucus Negra Flower Extract, PVP, Tannic Acid] marketed by Laboratoires Serobiologiques/Cognis, Liftline^{®} [INCI: Hydrolyzed wheat protein], Raffermine^{®} [INCI: Hydrolyzed Soy Flour] or Ridulisse C^{®} [Hydrolyzed Soy Protein] marketed by Silab, Serilesine^{®} [INCI: hexapeptide-10], Decorinyl^{™} [INCI: Tripeptide-10 Citrulline], Trylagen^{®} [INCI: Pseudoalteromonas Ferment Extract, Hydrolyzed Wheat Protein, Hydrolyzed Soy Protein, Tripeptide-10 Citrulline, Tripeptide-1], marketed by Lipotec, Ursolisome^{®} [INCI: Lecithin, Ursolic Acid, Atelocollagen, Xanthan Gum, Sodium Chondroitin Sulfate] or Collalift^{®} [INCI: Hydrolyzed Malt Extract] marketed by Coletica/Engelhard, Syn^{®}-Coll [INCI: Palmitoyl Tripeptide-5] marketed by Pentapharm, Hydriame^{®} [INCI: Water (Aqua), Glycosaminoglycans, Sclerotium Gum] marketed by Atrium Innovations or IP2000 [INCI: Dextran, Trifluoroacetyl tripeptide-2] marketed by Institut Europeen de Biologie Cellulaire among others.

In a particular embodiment, the anti-glycation agent is selected, for example and not restricted to, from the group formed by *Vaccinium angustifolium* extracts, ergothioneine and derivatives thereof, lysine, Aldenine^{®} [INCI: Hydrolized Wheat Protein, Hydrolized Soy Protein, Tripeptide-1], Vilastene^{™} [INCI: Lysine HCl, Lecithin, Tripeptide-10 Citrulline], dGlyage^{™} [INCI: Lysine HCl, Lecithin, Tripeptide-9 Citrulline] or Eyeseryl^{®} [INCI: Acetyl Tetrapeptide-5] marketed by Lipotec, hydroxystilbenes and derivatives thereof, resveratrol or 3,3',5,5'-tetrahydroxystilbene among others.

In a particular embodiment, the free radical scavenger and/or anti-atmospheric pollution agent, and/or the reactive carbonyl species scavenger is selected, for example and not restricted to, from the group formed by tea extract, olive leaf extract, *Rosmarinus officinalis* extract or *Eichhornia crassipes* extract, benzopyrenes, vitamin C and derivatives thereof, vitamin E and derivatives thereof, in particular tocopherol acetate, ascorbyl glycoside, phenols and polyphenols, in particular tannins, tannic acid and ellagic acid, gallocatechol, anthocyanins, chlorogenic acid, stilbenes, indoles, cysteine-containing amino acid derivatives, in particular *N*-acetylcysteine, ergothioneine, *S*-carboxymethylcysteine, chelating agents, in particular EDTA or ethylenediamines, carotenoids, bioflavonoids, ubiquinone, idebenone, catalase, superoxide dismutase, lactoperoxidase, glutathione peroxidase, glutathione, benzylidene camphor, pidolates, lignans, melatonin, oryzanol, carnosine and derivatives thereof, GHK [INCI: Tripeptide-1] and its salts and/or derivatives, Aldenine^{®} [INCI: Hydrolized wheat protein, hydrolized soy protein, tripeptide-1], Preventhelia^{™} [INCI: Diaminopropionoyl Tripeptide-33] or Lipochroman-6 [INCI: Dimethylmethoxy Chromanol] marketed by Lipotec, among others.

In a particular embodiment, the 5α-reductase inhibiting agent is selected, for example and not restricted to, from the group formed by extract of *Cinnamommum zeylanicum, Laminaria saccharina, Spiraea ulmaria, Nettle Root, Pygeum africanum, Avena Sativa, Serenoa repens,* extracts of the plants *Arnica montana, Cinchona succirubra, Eugenia caryophyllata, Humulus lupulus, Hypericum perforatum, Mentha piperita, Rosmarinus officinalis, Salvia officinalis, Thymus vulgaricus,* extract of plants of the genus *Silybum,* extract of plants which contain sapogenins and in particular extract of plants of the genus *Dioscorea,* retinoids and in particular retinol, sulfur and derivatives thereof, zinc salts and in particular lactate, gluconate, pidolate, carboxylate, salicylate or zinc cysteate, selenium chloride, vitamin B6, pyridoxine, capryloyl glycine, sarcosine, finasteride, dutasteride, izonsteride, turosteride and their salts, among others.

Likewise, in another particular embodiment, the lysyl- and/or prolyl-hydroxylase-inhibiting agent is selected, for example and not restricted to, from the group formed by 2,4-diaminopyrimidine 3-oxide or 2,4-diamino-6-piperidinopyrimidine 3-oxide, among others.

In another particular embodiment, the defensin synthesis-stimulating agent is selected, for example and not restricted to, from the group formed by extracts of or hydrolyzed *Aloe Vera, Roast amaranth, Rehmannias radix,* arnica, gardenia, carrot, orange, peach, pineapple, mint, gentian, hibiscus flower, walnut tree leaf, calabaza, peony, quinoa, boldo, rough bindweed, sunflower, elderberry, seaweed, hydrolyzed corn, hydrolyzed soy, hydrolyzed rice, valine and its isomers and derivatives, calcium and its salts, α-MSH and fragments contained in the amino acid sequence of α-MSH, vitamin A and its derivatives and precursors, vitamin D3 and its derivatives, jasmonic acid, fumaric acid, malic acid, citric acid, ascorbic acid, lactic acid, acetic acid, adipic acid, tartaric acid, cinnamic acid, glutamic acid, succinic acid, inulin, alkyl glucosides, poly-D-glutamic acid, glycine, L-methionine, L-alanine, L-citrulline, lactoprotein, casein, lactoperoxidase, lysozyme, polyphenol, alkyl glucosides, *Lactobacillus* extract, fusobacteria extracts or non-photosynthetic and unfruitful filamentous bacteria, acetyl-glutamyl-methionyl-alanyl-isoleucine, acetyl-arginyl-phenylglycyl-phenylglycine or acetyl-arginyl-6-aminohexanoyl-alanine marketed by Lipotec, among others.

In another particular embodiment, the bactericidal and/or bacteriostatic agent and/or antimicrobial and/or germicidal agent and/or fungicidal agent and/or fungistatic agent and/or germ inhibitor is selected, for example and not restricted to, from the group formed by macrolides, pyranosides, calcium channel blockers, for example and not restricted to, cinnarizine and diltiazem; hormones, for example and not restricted to, estril, analogues thereof or thyroxine and/or its salts, caprylyl glycol, imidazolidinyl urea, methyl 4-hydroxybenzoate [INCI: methylparaben], ethyl 4-hydroxybenzoate [INCI: ethylparaben], propyl 4-hydroxybenzoate [INCI: propylparaben], butyl 4-hydroxybenzoate [INCI: butylparaben], isobutyl 4-hydroxybenzoate [INCI: isobutylparaben], 1,3-bis(hydroxymethyl)-5,5-dimethylimidazolidine-2,4-dione [INCI: DMDM Hydantoin], benzyl 4-hydroxybenzoate [INCI: benzylparaben], benzyl alcohol, dehydroacetic acid, benzoic acid, sorbic acid, salicylic acid, formic acid, propionic acid, 2-bromo-2-nitropropane-1,3-diol, 3-*p*-chlorophenoxy-1,2-propanodiol [INCI: chlorphenesin], dichlorobenzyl alcohol, iodopropynyl butylcarbamate, benzalkonium chloride, odor-absorbing fungicides such as zinc ricinoleate, cyclodextrins, benzethonium chloride, chlorhexidine, ethanol, propanol, 1,3-butanediol, 1,2-propylene glycol, undecylenic acid, dehydroacetic acid, *N*-methylmorpholine acetonitrile (MMA), isopropanol, methanol, 1,2-hexanediol, 1,2-octanediol, pentylene glycol, glycerin laurate, glycerin caprilate, glycerin caprate, benzoyl peroxide, chlorhexidine gluconate, triclosan and derivatives thereof, phenoxyethanol, terpinen-4-ol, α-terpineol, resorcinol, stiemycin, erythromycin, neomycin, clindamycin and its esters, tetracyclines, metronidazole, azelaic acid, tolnaftate, nystatin, clotrimazole, ketoconazole, derivatives of zinc such as zinc piritionate or trithionate, zinc oxide and zinc undecylenate, piroctone olamine, isothiazolinones, selenium sulfur, benzyl hemiformal, boric acid, sodium borate, 6,6-dibromo-4,4-dichloro-2,2'-methylenediphenol [INCI: bromochlorophene], 5-bromo-5-nitro-1,3-dioxane, tosylchloramide sodium [INCI: chloramine T], chloroacetamide, *p*-chloro-*m*-cresol, 2-benzyl-4-chlorophenol [INCI: chlorophene], dimethyl oxazolidine, dodecyl dimethyl-2-phenoxyethyl ammonium bromide [INCI: domiphen bromide], 7-ethyl bicyclooxazolidine, hexetidine, glutaraldehyde, *N*-(4-chlorophenyl)-*N*-[4-chloro-3-(trifluoromethyl)phenyl]-urea [INCI: cloflucarban], 2-hydroxy-4-isopropyl-2,4,6-cycloheptatriene-1-one [INCI: Hinokitiol], isopropylmethylphenol, mercury salts, aluminum salts, nisin, phenoxyisopropanol, o-phenylphenol, 3-heptyl-2-[(3-heptyl-4-methyl-3*H*-thiazole-2-ylidene)methyl]-4-methylthiazole iodide [INCI: Quaternium-73], silver chloride, sodium iodide, thymol, undecylenic acid, diethylenetriaminepentaacetic acid, ethylenediaminetetraacetic acid and ethylenediaminetetraacetates, lactoperoxidase, glucose oxidase, lactoferrin, alkylaryl sulfonates, halogenated phenols, phenol mercury acetate and/or mixtures thereof, benzamidines, isothiazolines, derivatives of phthalimide, derivatives of pyridine, guanidines, quinolines, 1,2-dibromo-2,4-dicyanobutane, iodine-2-propylbutyl carbamate, iodine, tamed iodines, peroxo compounds, 4-chloro-3,5-dimethylphenol, 2,2'-methylene-bis(6-bromo-4-chlorophenol), 3-methyl-4-(1-methylethyl)phenol, 3-(4-chlorophenoxy)-1,2-propanediol, 3,4,4'-trichlorocarbanilide (TTC), thiamine essence, eugenol, farnesol, glycerin monolaurate, diglycerin monocaprinate, *N*-alkyl salicylic acid amides such as *n*-octyl salicylic acid amide or *n*-decyl salicylic acid amide, derivatives of halogenated xylene and cresol, such as *p*-chloro-meta-cresol or *p*-chloro-meta-xylene, extracts of *Allium sativum, Calendula officinalis, Chamomilla recutita, Echinacea Purpura, Hyssopus Officinalis, Melaleuca alternifolia* or tea tree oil, carnation essence, menthol and mint essence, among others.

Likewise, in another particular embodiment, the NO-synthase-inhibiting agent is selected, for example and not restricted to, from the group formed by extracts of the plants *Vitis vinifera, Olea europaea* or *Gingko biloba* among others.

In a particular embodiment, the desquamating agent and/or keratolytic agent and/or exfoliating agent is selected, for example and not restricted to, from the group formed by hydroxy acids and derivatives thereof, β-hydroxyacids, in particular salicylic acid and derivatives thereof, or gentisic acid; α-hydroxyacids and its salts, such as glycolic acid, ammonium glycolate, lactic acid, 2-hydroxyoctanoic acid, α-hydroxycaprylic acid, mandelic acid, citric acid, malic acid or tartaric acid; α- and β-hydroxybutyric acids; polyhydroxy acids such as gluconic acid, glucuronic acid or saccharic acid; keto acids such as pyruvic acid, glyoxylic acid; pyrrolidinecarboxylic acid; cysteic acid and derivatives; aldobionic acids; azelaic acid and derivatives thereof such as azeloyl diglycinate; ascorbic acid and derivatives thereof such as 6-*O*-palmitoylascorbic acid, ascorbyl glucoside, dipalmitoylascorbic acid, magnesium salt of ascorbic acid-2-phosphate (MAP), sodium salt of ascorbic acid-2-phosphate (NAP), ascorbyl tetraisopalmitate (VCIP); nicotinic acid, its esters and nicotinamide (also called vitamin B3 or vitamin PP); nordihydroguaiaretic acid; urea; oligofucoses; cinnamic acid; derivatives of jasmonic acid; hydroxy stilbenes such as resveratrol; *Saccarum officinarum* extract; enzymes involved in desquamation or degradation of the corneodesmosomes, such as glycosidases, stratum corneum chymotryptic enzyme (SCCE) or other proteases such as trypsin, chymotrypsin, sutilain, papain or bromelain; chelating agents such as ethylenediaminetetraacetic acid (EDTA), aminosulfonic compounds such as 4-(2-hydroxyethyl)piperazine-1-ethanesulfonic acid (HEPES) or sodium methylglycine diacetate (TRILON^{®} M marketed by BASF); derivatives of 2-oxothiazolidine-4-carboxylic acid (procysteine); derivatives of sugars such as *O-*octanoyl-6-D-maltose and *N*-acetylglucosamine; chestnut extract *(Castanea sativa)* such as that marketed by SILAB under the name Recoverine^{®} [INCI: Water (Aqua), Castanea Sativa Seed Extract]; opuntia extract *(Opuntia ficus-indica)* such as that marketed by SILAB as Exfolactive^{®} [INCI: Hydrolyzed Opuntia Ficus Indica Flower Extract]; or Phytosphingosine SLC^{®} [INCI: Salicyloyl Phytosphingosine] marketed by Degussa/Evonik, Peel-Moist [INCI: Glycerin, Papain, Calcium Pantothenate, Xanthan Gum, Caprylyl Glycol, Urea, Magnesium Lactate, Ethylhexylglycerin, Potassium Lactate, Serine, Alanine, Proline, Magnesium Chloride, Sodium Citrate]; extract or combination of extracts of *Saphora japonica,* papaya, pineapple, squash or yam, and/or mixtures thereof.

In another particular embodiment, the anti-inflammatory agent and/or analgesic agent is selected, for example and not restricted to, from the group formed by madecassoside extract, echinacea extract, amaranth seed oil, sandal wood oil, peach tree leaf extract, extract of *Aloe vera, Arnica montana, Artemisia vulgaris, Asarum maximum, Calendula officinalis, Capsicum, Centipeda cunninghamii, Chamomilla recutita, Crinum asiaticum, Hamamelis virginiana, Harpagophytum procumbens, Hypericum perforatum, Lilium candidum, Malva sylvestris, Melaleuca alternifolia, Origanum majorana, Origanum vulgare, Prunus laurocerasus, Rosmarinus officialis, Salix alba, Silybum marianum, Tanacetum parthenium, Thymus vulgaris, Uncaria guianensis* or *Vaccinum myrtillus,* mometasone furoate, prednisolone, nonsteroidal antiinflammatories including cyclooxygenase or lipoxygenase inhibitors, benzydamine, acetylsalicylic acid, rosmarinic acid, ursolic acid, derivatives of glycyrrhizinate, α-bisabolol, azulene and analogues, sericoside, ruscogenin, escin, scoline, rutin and analogues, hydrocortisone, clobetasol, dexamethasone, prednisone, paracetamol, amoxiprin, benorilate, choline salicylate, faislamine, methyl salicylate, magnesium salicylate, salsalate, diclofenac, aceclofenac, acemetacin, bromfenac, etodolac, indomethacin, oxamethacin, proglumetacin, sulindac, tolmetin, ibuprofen, dexibuprofen, carprofen, fenbufen, fenoprofen, flurbiprofen, ketoprofen, dexketoprofen, ketorolac, loxoprofen, naproxen, miroprofen, oxaprozin, pranoprofen, tiaprofenic acid, suprofen, mefenamic acid, meclofenamate, meclofenamic acid, flufenamic acid, tolfenamic acid, nabumetone, phenylbutazone, azapropazone, clofezone, kebuzone, metamizole, mofebutazone, oxyphenbutazone, phenazone, sulfinpyrazone, piroxicam, lornoxicam, meloxicam, tenoxicam, celecoxib, etoricoxib, lumiracoxib, parecoxib, rofecoxib, valdecoxib, nimesulide, naproxcinod, fluproquazone or licofelone, omega-3 and omega-6 fatty acids, morphine, codeine, oxycodone, hydrocodone, diamorphine, pethidine, tramadol, bupenorphine, benzocaine, lidocaine, chloroprocaine, tetracaine, procaine, amitriptyline, carbamazepine, gabapentin, pregabalin, bisabolol, Neutrazen^{™} [INCI: Water, Butylene Glycol, Dextran, Palmitoyl Tripeptide-8] marketed by Atrium Innovations/Unipex Group, Meliprene^{®} [INCI: Dextran, Acetyl Heptapeptide-1] marketed by Institut Européen de Biologie Cellulaire/Unipex Group, Skinasensyl^{™} [INCI: Acetyl Tetrapeptide-15] or Anasensyl^{™} [INCI: Mannitol, Ammonium Glycyrrhizate, Caffeine, Hippocastanum (Horse Chestnut) Extract] marketed by Laboratoires Serobiologiques/Cognis, Calmosensine^{™} [INCI: Acetyl Dipeptide-1] marketed by Sederma, coenzyme Q10 or alkylglycerine ethers.

In addition, in another particular embodiment, the whitening or depigmenting agent is selected, for example and not restricted to, from the group formed by extracts of *Achillea millefolium, Aloe vera, Aradirachta indica, Asmuna japonica, Autocarpus incisus, Bidens pilosa, Broussonetia papyrifera, Chlorella vulgaris, Cimicifuga racemosa,* Emblica officinalis, *Glycyrrhiza glabra, Glycyrrhiza uralensis, Ilex purpurea, Ligusticum lucidum, Ligusticum wallichii, Mitracarpus scaber, Morinda citrifolia, Morus alba, Morus bombycis, Naringi crenulata, Prunus domesticus, Pseudostellariae radix, Rumex crispus, Rumex occidentalis, Sapindus mukurossi, Saxifragia sarmentosa, Scutellaria Galericulate, Sedum sarmentosum Bunge, Stellaria medica, Triticum Vulgare, Uva ursi* or *Whitania somnifera,* flavonoides, soy extract, lemon extract, orange extract, ginkgo extract, cucumber extract, geranium extract, gayuba extract, carob extract, cinnamon extract, marjoram extract, rosemary extract, clove extract, soluble liquorice extract or blackberry leaf extract, Lipochroman-6 [INCI: Dimethylmethoxy Chromanol] or Chromabright^{™} [INCI: Dimethylmethoxy Chromanyl Palmitate] marketed by Lipotec, Actiwhite^{™} LS9808 [INCI: Aqua, Glycerin, Sucrose Dilaurate, Polysorbate 20, Pisum sativum (Pea) extract] or Dermawhite^{®} NF LS9410 [INCI: Mannitol, Arginine HCl, Phenylalanine, Disodium EDTA, Sodium Citrate, Kojic Acid, Citric Acid, Yeast Extract] marketed by Laboratoires Serobiologiques/Cognis, Lumiskin^{™} [INCI: Caprylic/Capric Triglycerid, Diacetyl-Boldine], Melaclear^{™} [INCI: Glycerin, Aqua, Dithiaoctanediol, Gluconic acid, Sutilains, Beta-carotene], O.D.A.white^{™} [INCI: octadecendioic acid] or Etioline^{™} [INCI: Glycerin, Butylene Glycol, Arctostaphylos uva ursi Leaf Extract, Mitracarpus scaber Extract] marketed by Sederma, Sepiwhite^{™} MSH [INCI: Undecylenoyl Phenylalanine] marketed by Seppic, Achromaxyl [INCI: Aqua, Brassica napus Extract] marketed by Vincience, Gigawhite^{™} [INCI: Aqua, Glycerin, Malva sylvestris (Mallow) Extract, Mentha piperita Leaf Extract, Primula veris Extract, Alchemilla vulgaris Extract, Veronica officinalis Extract, Melissa officinalis Leaf Extract, Achillea millefolium Extract], Melawhite^{®} [INCI: Leukocyte Extract, AHA] or Melfade^{®}-J [INCI: Aqua, Arctostaphylos uva-ursi Leaf Extract, Glycerin, Magnesium Ascorbyl Phosphate] marketed by Pentapharm, Albatin^{®} [INCI: Aminoethylphosphoric Acid, Butylene Glycol, Aqua] marketed by Exsymol, Tyrostat^{™}-11 [INCI: Aqua, Glycerin, Rumex occidentalis Extract] or Melanostatine^{®}-5 [INCI: Dextran, Nonapeptide-1] marketed by Atrium Innovations, arbutin and its isomers, kojic acid and derivatives thereof, ascorbic acid and derivatives thereof such as 6-*O-*palmitoylascorbic acid, ascorbyl glucoside, dipalmitoylascorbic acid, magnesium salt of ascorbic acid-2-phosphate (MAP), sodium salt of ascorbic acid-2-phosphate (NAP), ascorbyl glucoside or ascorbyl tetraisopalmitate (VCIP); retinol and derivatives thereof including tretinoin and isotretinoin, idebenone, hydroxybenzoic acid and derivatives thereof, niacinamide, liquiritin, resorcinol and derivatives thereof, hydroquinone, α-tocopherol, γ-tocopherol, azelaic acid, azeloyl diglycinate, resveratrol, linoleic acid, α-lipoic acid, dihydrolipoic acid, α-hydroxy acids, β-hydroxy acids, ellagic acid, ferulic acid, cinnamic acid, oleanolic acid, aloesin and its derivatives and/or serine protease inhibitors, for example and not restricted to, tryptase, trypsin or PAR-2 inhibitors, among others.

In another particular embodiment, the melanin synthesis stimulating agent, propigmenting agent, self-tanning agent and/or melanocyte proliferation stimulating agent is selected, for example and not restricted to, from the group formed by extracts of *Citrus Aurantium Dulcis Fruit, Coleus forskohlii, Coleus Esquirolii, Coleus Scutellariodes, Coleus Xanthanthus, Ballota nigra, Ballota lanata, Ballota suavelens, Marrubium cylleneum, Cistus creticus, Amphiachyris amoena, Aster oharai, Otostegia fruticosa, Plectranthus barbatus, Halimium viscosum* or *Larix laricema,* dihydroxyacetone and derivatives thereof, sugars, for example and not restricted to, erythrulose, melanin and derivatives thereof including melanin polymers and derivatives of melanin with a low molecular weight which are soluble in water, forskolin and derivatives thereof including deacetylforskolin and isoforskolin, tyrosine and derivatives thereof including acetyl tyrosine, oleoyl tyrosine, 3-amino tyrosine and 3-nitrotyrosine, copper salts such as CuCl₂, carotenoids, canthaxanthins, polymers of dihydroxyindole carboxylic acid, 3,4-dihydroxybenzoic acid, 3-amino-4-hydroxybenzoic acid, aloin, emodin, alizarin, dihydroxyphenylalanine, 4,5-dihydroxynaphthalene-2-sulfonic acid, 3-dimethylaminophenol or *p*- aminobenzoic acid, Melatime^{™} [INCI: Acetyl Tripeptide-40] marketed by Lipotec, Heliostatine IS^{™} [INCI: Pisum Sativum Extract] marketed by Vincience/ISP, Vegetan [INCI: Dihydroxyacetone] or Vegetan Premium [INCI: Dihydroxyacetone, Melanin] marketed by Soliance, MelanoBronze [INCI: Vitex Agnus Castus Extract, Acetyl Tyrosine] marketed by Mibelle Biochemistry, Melitane^{®} [INCI: Acetyl Hexapeptide-1] marketed by Institut Europeen de Biologie Cellulaire/Unipex Innovations, Actibronze^{®} [INCI: Hydrolyzed Wheat Protein, Acetyl Tyrosine, Copper Gluconate] or Instabronze^{®} [INCI: Dihydroxyacetone, Tyrosine] marketed by Alban Muller, Thalitan [INCI: Hydrolyzed Algin, Magnesium Sulfate, Manganese Sulfate] marketed by CODIF, Tyrosilane^{®} [INCI: Methylsilanol Acetyltyrosine] marketed by Exsymol, Tyr-Excel^{™} [INCI: Oleoyl Tyrosine, Luffa Cylindrica Seed Oil, Oleic Acid] or Tyr-Ol [INCI: Oleoyl Tyrosine, Butylene glycol, Oleic Acid] marketed by Sederma/Croda, Bronzing S.F. [proposed INCI: Butiryl Pentapeptide] marketed by Infinitec Activos or Biotanning^{®} [INCI: Hydrolyzed Citrus Aurantium Dulcis Fruit Extract] marketed by Silab, among others.

In a particular embodiment, the anti-wrinkle and/or anti-aging agent is selected, for example and not restricted to, from the group formed by extracts of *Vitis vinifera, Rosa canina, Curcuma longa, Iris pallida, Theobroma cacao, Ginkgo biloba, Leontopodium Alpinum* or *Dunaliella salina,* Matrixyl^{®} [INCI: Palmitoyl Pentapeptide-4], Matrixyl 3000^{®} [INCI: Palmitoyl Tetrapeptide-7, Palmitoyl Oligopeptide], Essenskin^{™} [INCI: calcium hydroxymethionine], Renovage [INCI: teprenone] or Dermaxyl^{®} [INCI: Palmitoyl Oligopeptide] marketed by Sederma, Vialox^{®} [INCI: Pentapeptide-3], Syn^{®}-Ake^{®} [INCI: Dipeptide Diaminobutyroyl Benzylamide Diacetate], Syn^{®}-Coll [INCI: Palmitoyl Tripeptide-5], Phytaluronate [INCI: Locust Bean (Ceratonia Siliqua) Gum] or Preregen^{®} [INCI: Glycine Soja (Soybean) Protein, Oxido Reductases] marketed by Pentapharm/DSM, Myoxinol^{™} [INCI: Hydrolyzed Hibiscus Esculentus Extract], Syniorage^{™} [INCI: Acetyl Tetrapeptide-11], Dermican^{™} [INCI: Acetyl Tetrapeptide-9] or DN-AGE^{™} LS [INCI: Cassia Alata leaf Extract] marketed by Laboratoires Sérobiologiques/Cognis, Algisum C^{®} [INCI: Methylsilanol Mannuronate] or Hydroxyprolisilane CN^{®} [INCI: Methylsilanol Hydroxyproline Aspartate] marketed by Exsymol, Argireline^{®} [INCI: Acetyl Hexapeptide-8], SNAP-7 [INCI: Acetyl Heptapeptide-4], SNAP-8 [INCI: Acetyl Octapeptide-3], Leuphasyl^{®} [INCI: Pentapeptide-18], Aldenine^{®} [INCI: Hydrolized wheat protein, hydrolized soy protein, Tripeptide-1], Preventhelia^{™} [INCI: Diaminopropionoyl Tripeptide-33], Decorinyl^{™} [INCI: Tripeptide-10 Citrulline], Trylagen^{®} [INCI: Pseudoalteromonas Ferment Extract, Hydrolyzed Wheat Protein, Hydrolyzed Soy Protein, Tripeptide-10 Citrulline, Tripeptide-1], Eyeseryl^{®} [INCI: Acetyl Tetrapeptide-5], Peptide AC29 [INCI: Acetyl Tripeptide-30 Citrulline], Lipochroman-6 [INCI: Dimethylmethoxy Chromanol], Chromabright^{™} [INCI: Dimethylmethoxy Chromanyl Palmitate], Antarcticine^{®} [INCI: Pseudoalteromonas Ferment Extract], Vilastene^{™} [INCI: Lysine HCl, Lecithin, Tripeptide-10 Citrulline], dGlyage^{™} [INCI: Lysine HCl, Lecithin, Tripeptide-9 Citrulline], acetyl-arginyl-phenylglycyl-tryptophyl-phenylglycine, acetyl-arginyl-phenylglycyl-valyl-glycine or acetyl-arginyl-phenylglycyl-valyl-phenylglycine, Inyline^{™} [INCI: Acetyl Hexapeptide-30] marketed by Lipotec, Kollaren^{®} [INCI: Tripeptide-1, Dextran] marketed by Institut Europeen de Biologie Cellulaire, Collaxyl^{®} IS [INCI: Hexapeptide-9], Laminixyl IS^{™} [INCI: Heptapeptide], Orsirtine^{™} GL [INCI: Oryza Sativa (Rice) Extract], D'Orientine^{™} IS [INCI: Phoenix Dactylifera (Date) Seed Extract], Phytoquintescine^{™} [INCI: Einkorn (Triticum Monococcum) Extract] or Quintescine^{™} IS [INCI: Dipeptide-4] marketed by Vincience/ISP, BONT-L-Peptide [INCI: Palmitoyl Hexapeptide-19] marketed by Infinitec Activos, Deepaline^{™} PVB [INCI: Palmitoyl hydrolyzed Wheat Protein] or Sepilift^{®} DPHP [INCI: Dipalmitoyl Hydroxyproline] marketed by Seppic, Gatuline^{®} Expression [INCI: Acmella oleracea Extract], Gatuline^{®} In-Tense [INCI: Spilanthes Acmella Flower Extract] or Gatuline^{®} Age Defense 2 [INCI: Juglans Regia (Walnut) Seed Extract] marketed by Gattefossé, Thalassine^{™} [INCI: Algae Extract] marketed by Biotechmarine, ChroNOline^{™} [INCI: Caprooyl Tetrapeptide-3] or Thymulen-4 [INCI: Acetyl Tetrapeptide-2] marketed by Atrium Innovations/Unipex Group, EquiStat [INCI: Pyrus Malus Fruit Extract, Glycine Soja Seed Extract] or Juvenesce [INCI: Ethoxydiglicol and Caprylic Triglycerid, Retinol, Ursolic Acid, Phytonadione, Ilomastat] marketed by Coletica, Ameliox [INCI: Carnosine, Tocopherol, Silybum Marianum Fruit Extract] or PhytoCellTec Malus Domestica [INCI: Malus Domestica Fruit Cell Culture] marketed by Mibelle Biochemistry, Bioxilift [INCI: Pimpinella Anisum Extract] or SMS Anti-Wrinkle^{®} [INCI: Annona Squamosa Seed Extract] marketed by Silab, Ca²⁺ channel blockers, for example and not restricted to, alverin, manganese or magnesium salts, certain secondary or tertiary amines, retinol and derivatives thereof, resveratrol, idebenone, coenzyme Q10 and derivatives thereof, boswellic acid and derivatives thereof, GHK and derivatives thereof and/or salts, carnosine and derivatives thereof, DNA repair enzymes, for example and not restricted to, photolyase or T4 endonuclease V, or chloride channel blockers among others.

In a particular embodiment, the lipolytic agent or agent stimulating lipolysis, venotonic agent and/or anti-cellulite agent is selected, for example and not restricted to, from the group formed by extracts of *Bupleurum Chinensis, Cecropia Obtusifolia, Celosia Cristata, Centella Asiatica, Chenopodium Quinoa, Chrysanthellum Indicum, Citrus Aurantium Amara, Coffea Arabica, Coleus Forskohlii, Commiphora Myrrha, Crithmum Maritimum, Eugenia Caryophyllus, Ginkgo Biloba, Hedera Helix* (ivy extract), *Hibiscus Sabdariffa, Ilex Paraguariensis, Laminaria Digitata, Nelumbium Speciosum, Paullinia Cupana, Peumus Boldus, Phyllacantha Fibrosa, Prunella Vulgaris, Prunus Amygdalus Dulcis, Ruscus Aculeatus* (Butcherbroom extract), *Sambucus Nigra, Spirulina Platensis Algae, Uncaria Tomentosa* or *Verbena Officinalis,* dihydromyricetin, coenzyme A, lipase, glaucine, esculin, visnadine, Regu^{®}-Shape [INCI: Isomerized Linoleic Acid, Lecithin, Glycerin, Polysorbate 80] marketed by Pentapharm/DSM, UCPeptide^{™} V [INCI: Pentapeptide] or AT Peptide^{™} IS [INCI: Tripeptide-3] marketed by Vincience/ISP, Liporeductyl^{®} [INCI: Caffeine, Butcherbroom (Ruscus Aculeatus) Root Extract, TEA-Hydroiodide, Carnitine, Ivy (Hedera Helix) Extract, Escin, Tripeptide-1] marketed by Lipotec, Adiposlim [INCI: Sorbitan Laurate, Lauroyl Proline] marketed by SEPPIC, caffeine, carnitine, escin and/or triethanolamine iodide, among others.

In a particular embodiment, the heat shock protein synthesis stimulating agent is selected, for example and not restricted to, from the group formed by extracts of *Opuntia ficus indica, Salix alba, Lupinus spp., Secale cereale,* extracts of red algae from the genus *Porphyra,* extracts of crustaceans from the genus *Artemia,* jojoba seed oil, grape seed extracts, green tea extracts, geranylgeranylacetone, celastrol, zinc and its salts, 2-cyclopenten-1-one, proteasome inhibitors, for example and not restricted to, bortezomib; prostaglandins and derivatives thereof, hydroxylamine and derivatives thereof, for example and not restricted to, bimoclomol; chalcone and derivatives thereof, hyperosmotic agents, for example and not restricted to, sorbitol and derivatives thereof, mannitol and derivatives thereof or glycerol and derivatives thereof, isosorbide, urea or salicylic acid and derivatives thereof among others, or mixtures thereof.

In a particular embodiment, the hair growth inducing or hair loss retardant agent is selected, for example and not restricted to, from the group formed by the extracts of *Tussilago farfara* or *Achillea millefolium,* nicotinic acid esters such as C₃-C₆ alkyl nicotinates such as methyl or hexyl nicotinate, benzyl nicotinate, or tocopheryl nicotinate; biotin, 5α-reductase-inhibiting agents, anti-inflammatory agents, retinoids, for example and not restricted to, All-trans-retinoic acid or tretinoin, isotretinoin, retinol or vitamin A, and derivatives thereof, such as acetate, palmitate, propionate, motretinide, etretinate and zinc salt of trans-retinoic acid; anti-bacterial agents, calcium channel blockers, for example and not restricted to, cinnarizine and diltiazem; hormones, for example and not restricted to, estriol, its analogues or thyroxine, its analogues and/or salts; antiandrogenic agents, for example and not restricted to, oxendolone, spironolactone or diethylstilbestrol; anti-radical agents, esterified oligosaccharides, for example and not restricted to, those described in documents EP 0211610 and EP 0064012; derivatives of hexasaccharide acids, for example and not restricted to, glucose-saccharide acid or those described in document EP 0375388; glucosidase inhibitors, for example and not restricted to, D-glucaro-1,5-lactam or those described in document EP 0334586; glycosaminoglycanase and proteoglycanase inhibitors, for example and not restricted to L-galactono-1,4-lactone or those described in document EP 0277428; tyrosine kinase inhibitors, for example and not restricted to, 1-amido-1-cyano(3,4-dihydroxyphenyl)ethylene or those described in document EP 0403238, diazoxides, for example and not restricted to, 7-(acetylthio)-4',5'-dihydrospiro[androst-4-ene-17,2'-(3H)furan]-3-one, 1,1-dioxide of 3-methyl-7-chloro[2H]-1,2,4-benzothiadiazine or spirooxazine; phospholipids, for example and not restricted to, lecithin; salicylic acid and derivatives thereof, hydroxycarboxylic or keto carboxylic acid and esters thereof, lactones and their salts; anthralin, eicose-5,8,11-trienoic acids and esters thereof or amides among others, minoxidil and derivatives thereof or mixtures thereof.

In another particular embodiment the body hair growth inhibiting or retardant agent is selected, for example and not restricted to, from the group formed by activin or activin agonists, flavonoids such as quercetin, curcumin, galangin, fisetin, myricetin, apigenin; propyl gallate, nordihydroguaiaretic acid, caffeic acid, tyrosine kinase inhibitors such as lavendustin, erbstatin, tyrphostins, benzoquinone-ansamycin herbimycin A, thiazolidinediones, phenazocine, 2,3-dihydro-2-thioxo-1H-indol-3-alcanoic acids, phenothiazine derivatives such as thioridazine; sphingosine and derivatives thereof, staurosporine and derivatives thereof, glycyrrhetinic acid, lauryl isoquinolinium bromide, Decelerine^{™} [INCI: Lauryl Isoquinolium Bromide, Pseudoalteromonas Ferment Extract] marketed by Lipotec or serine protease inhibitors, trypsin and/or mixtures thereof.

In a particular embodiment, the cosmetic and/or absorbent and/or body odor masking deodorant and/or antiperspirant agent, perfuming substance and/or perfumed oil is selected, for example and not restricted to, from the group formed by the complex zinc salt of ricinoleic acid, Styrax, derivatives of abiotic acid, sage essence, chamomile essence, carnation essence, lemon balm essence, mint essence, cinnamon leaf essence, lime flower essence, juniper berry essence, vetiver essence, olibanum essence, galbanum essence, labdanum essence, lavender essence, peppermint essence, bergamot orange, dihydromyrcenol, lilial, lyral, citronellol, lemon essence, mandarin essence, orange essence, lavender essence, muscat, geranium bourbon essence, aniseed, cilantro, cumin, juniper, extracts of fleur-de-lis, lilac, roses, jasmin, bitter orange blossom; benzyl acetate, *p*-*tert*-butylcyclohexyl acetate, linalyl acetate, phenylethyl acetate, ethylmethylphenyl glycinate, linalyl benzoate, benzyl formate, allyl cyclohexyl propionate, styrallyl propionate, benzyl salicylate, benzyl ethyl ether, linear alkanes with from 8 to 18 carbon atoms, citral, ricinoleic acid, citronellal, citronellyl oxyacetaldehyde, cyclamen aldehyde, hydroxycitronellal, bourgeonal, ionones, methyl cedryl ketone, anethole, eugenol, isoeugenol, geraniol, linalool, terpineol, phenylethyl alcohol, α-hexylcinnamaldehyde, geraniol, benzylacetone, cyclamen aldehyde, Boisambrene Forte^{®}, ambroxan, indole, hedione, sandelice, cyclovertal, β-damascone, allyl amyl glycolate, dihydromyrcenol, phenoxyethyl isobutyrate, cyclohexyl salicylate, Vertofix Coeur, Iso-E-Super, Fixolide NP, Evernyl, phenylacetic acid, geranyl acetate, romillat, irotyl, floramate, active astringent products such as aluminum chloride, aluminum chlorohydrate, aluminum dichlorohydrate, aluminum sesquichlorohydrate, aluminum hydroxyallantoinate, aluminum chlorotartrate, aluminum and zirconium trichlorohydrate, aluminum and zirconium tetrachlorohydrate, aluminum and zirconium pentachlorohydrate and/or mixtures thereof.

In a particular embodiment, the antioxidant is selected, for example and not restricted to, from the group formed by butylhydroxytoluene (BHT), butylhydroxyanisole (BHA), tert-butylhydroquinone (TBHQ), 2,6,-di-tert-butyl-4-methylphenol, gallic acid esters such as propyl gallate, probucol, polyphenoles, ascorbic acid and its salts, enzymes such as catalase, superoxide dismutase and peroxidases; citric acid, citrates, monoglyceride esters, calcium metabisulfate, lactic acid, malic acid, succinic acid, tartaric acid, vitamin A or β-carotene, vitamins E and C, tocopherols such as vitamin E acetate, ascorbic acid esters such as ascorbyl palmitate and ascorbyl acetate, zinc, copper, mannitol, reduced glutathione, carotenoids such as cryptoxanthin, astaxanthin and lycopene; cysteine, uric acid, carnitine, taurine, tyrosine, lutein, zeaxanthin, N-acetyl-cysteine, carnosine, γ-glutamylcysteine, quercetin, lactoferrin, dihydrolipoic acid, tea catechins, retinyl palmitate and derivatives thereof, bisulfate, metabisulfite and sodium sulfite, chromans, chromens and their analogues, Lipochroman-6 [INCI: Dimethylmethoxy Chromanol], chelating agents of metals such as EDTA, sorbitol, phosphoric acid or dGlyage^{™} [INCI: Lysine HCl, Lecithin, Tripeptide-9 Citrulline]; extract of *Ginkgo Biloba,* plant extracts such as sage, pomegranate, rosemary, oregano, ginger, marjoram, cranberry, grape, tomato, green tea or black tea; oleoresin extract, extract of plants which contain phenols such as vanillin, ellagic acid and resveratrol; tertiary butylhydroquinone or mixtures thereof, metal salts with a valence of 2 such as selenium, cadmium, vanadium or zinc; α-lipoic acid, coenzyme Q, idebenone or derivatives thereof.

In a particular embodiment, the agent inhibiting sweat-degrading enzymes is selected, for example and not restricted to, from the group formed by trialkyl citrates such as trimethyl citrate, tripropyl citrate, triisopropyl citrate, tributyl citrate or triethyl citrate; lanosterine sulfate or phosphate, cholesterin, campesterin, stigmasterin and sitosterin; dicarboxylic acids and their esters, such as glutaric acid, monoethyl glutarate, diethyl glutarate, adipic acid, monoethyl adipate, diethyl adipate; malonic acid and diethyl malonate, hydroxycarboxylic acids and their esters such as malic acid, tartaric acid or diethyl tartrate, zinc glycinate and/or mixtures thereof.

In another particular embodiment, the agent capable of filtering UV rays is selected, for example and not restricted to, from the group formed by organic or mineral photoprotective agents active against A and/or B ultraviolet rays such as substituted benzotriazoles, substituted diphenylacrylates, organic nickel complexes, umbelliferone, urocanic acid, biphenyl derivatives, stilbene, 3-benzylidene camphor, and derivatives thereof such as 3-(4-methylbenzylidene)camphor; derivatives of 4-aminobenzoic acid, 2-ethylhexyl 4-(dimethylamino)benzoate, 2-octyl 4-(dimethylamino)benzoate and amyl 4-(dimethylamino)benzoate; cinnamic acid esters, such as 2-ethylhexyl 4-methoxycinnamate or diethylamino hydroxybenzoyl hexyl benzoate, propyl 4-methoxycinnamate, isoamyl 4-methoxycinnamate, 2-ethylhexyl (octocrylenes) 2-cyano-3,3-phenyl cinnamate; salicylic acid esters, such as 2-ethylhexyl salicylate, 4-isopropylbenzyl salicylate, homomenthyl salicylate; benzophenone derivatives, such as 2-hydroxy-4-methoxybenzophenone, 2-hydroxy-4-methoxy-4'-methylbenzophenone, 2,2'-dihydroxy-4-methoxybenzophenone; benzalmalonic acid esters, such as di-2-ethylhexyl 4-methoxybenzalmalonate; triazine derivatives, such as 2,4,6-trianilino, p-carbo-2'-ethyl-1'-hexyloxy-1,3,5-triazine, octyl triazone or dioctyl butamido triazones; propane-1,3-diones, such as 1-(4-*tert*-butylphenyl)-3-(4'-methoxyphenyl)propane-1,3-dione; ketotricyclo(5.2.1.0)decane derivatives; 2-phenylbenzimidazole-5-sulfonic acid; benzophenone sulfonic acid derivatives, such as 2-hydroxy-4-methoxybenzophenone-5-sulfonic acid and its salts; 4-(2-oxo-3-bornylidenemethyl)benzenesulfonic acid, benzoyl methane derivatives, such as benzoyl methane 2-methyl-5-(2-oxo-3-bornylidene)sulfonic acid, such as 1-(4'-*tert*-butylphenyl)-3-(4'-methoxyphenyl)propane-1,3-dione, 4-*tert*-butyl-4'-methoxydibenzoylmethane, 1-phenyl-3-(4'-isopropylphenyl)-propane-1,3-dione, enamine compounds, anthranilates, silicons, benzimidazole derivatives, imidazolines, benzoyl derivatives, Chromabright^{™} [INCI: Dimethylmethoxy Chromanyl Palmitate] or Preventhelia^{™} [INCI: Diaminopropionoyl Tripeptide-33] both marketed by Lipotec, metal oxides such as zinc oxide, titanium, iron, zirconium, silicon, manganese, aluminum and cerium; silicates, talc, barium sulfate, zinc stearate, carbon nanotubes and/or mixtures thereof.

In addition, in another particular embodiment, the agent stimulating or regulating keratinocyte differentiation is selected, for example and not restricted to, from the group formed by minerals such as calcium, retinoids such as retinol or tretinoin, analogues of vitamin D3 such as calcitriol, calcipotriol or tacalcitol, lupine *(Lupinus albus)* extract such as that marketed by SILAB under the name Structurin^{®} [INCI: Hydrolyzed Lupine Protein], β-sitosterol sulfate, such as that marketed by Vincience/ISP under the name Phytocohesine PSP^{®} [INCI: Sodium Beta-sitosterol Sulfate], maize *(Zea Mays)* extract such as that marketed by Solabia under the name Phytovityl C^{®} [INCI: Water (Aqua), Zea Mays Extract], Helix Aspersa Müller glycoconjugates and/or mixtures thereof.

Likewise, in another particular embodiment, the muscle relaxant, agent inhibiting muscle contraction, agent inhibiting acetylcholine receptor clustering and/or anticholinergic agent is selected, for example and not restricted to, from the group formed by extracts of *Atropa belladonna, Hyoscyamus niger, Mandragora officinarum, Chondodendron tomentosum,* plants of the *Brugmansia* genus, or the *Datura* genus, *Clostridium botulinum* toxin, peptides derived from the protein SNAP-25 or Inyline^{™} [INCI: Acetyl Hexapeptide-30] marketed by Lipotec, baclofen, carbidopa, levodopa, bromocriptine, chlorphenesin, chlorzoxazone, donepezil, mephenoxalone, reserpine, tetrabenazine, dantrolene, thiocolchicoside, tizanidine, clonidine, procyclidine, glycopyrrolate, atropine, hyoscyamine, benztropine, scopolamine, promethazine, diphenhydramine, dimenhydrinate, dicyclomine, cyclobenzaprine, orphenadrine, flavoxate, cyclopentolate, ipratropium, oxybutynin, pirenzepine, tiotropium, trihexyphenidyl, tolterodine, tropicamide, solifenacin, darifenacin, mebeverine, trimethaphan, atracurium, cisatracurium, doxacurium, fazadinium, metocurine, mivacurium, pancuronium, pipecuronium, rapacuronium, tubocuranine, dimethyl tubocuranine, rocuronium, vecuronium, suxamethonium, 18-methoxycoronaridine, carisoprodol, febarbamate, meprobamate, metocarbamol, phenprobamate, tibamate, anticonvulsant agents such as levetiracetam, stiripentol, phenobarbital, methylphenobarbital, pentobarbital, metharbital, barbexaclone, pirimidone, carbamazepine, oxcarbazepine, benzodiazepines, for example and not restricted to, clonazepam, cloxazolam, clorazepate, diazepam, flutoprazepam, lorazepam, midazolam, nitrazepam, nimetazepam, phenazepam, temazepam, tetrazepam or clobazam, among others.

In another particular embodiment, the active ingredient of the delivery system of this invention is selected from the group formed by pharmaceutical active ingredients and/or adjuvants.

In particular, the pharmaceutical active ingredients and/or adjuvants are selected, for example and not restricted to, from the group formed by antiacids, agents against peptic ulcers and gastroesophageal reflux disease, antispasmodics, analgesics, anticholinergic drugs, propulsive drugs, antiemetics, antinausea drugs, agents for biliary therapy, agents for hepatic therapy, lipotropics, laxatives, antidiarrhetics, intestinal adsorbents, antipropulsives, anti-inflammatory drugs, active ingredients against obesity, enzymes, hypoglycemic drugs, insulin and analogues, vitamins, proteins, minerals, anabolic steroids, antithrombotic agents, antifibrinolytics, haemostatic agents, antiarrhythmic agents, cardiac stimulants, cardiac glycosides, vasodilators, antiadrenergic agents, antihypertensive drugs, diuretics, potassium-saving agents, antihemorrhoidals, antivaricose therapy agents, capillary stabilizing agents, agents which act on the renin-angiotensin system, beta-blockers, selective calcium-channel blockers, non-selective calcium-channel blockers, ACE inhibitors, angiotensin II inhibitors, agents modifying lipids, antifungals, healing agents, antipruritics, antihistamines, anesthetics, antipsoriatics, chemotherapy drugs, corticosteroids, antiseptics, disinfectants, anti-acne agents, products for gynecological use, oxytocics, anticonceptives, androgen, estrogen, progestagen, ovulation stimulants, gonadotropins, antiandrogens, products for urological use, antispasmodics, drugs used in benign prostatic hypertrophy, hormones, hormone antagonists, antibiotics, tetracyclines, anphenicols, beta-lactam antibacterials, penicillin, sulfonamides, trimethoprim, macrolides, lincosamides, streptogramins, antibacterial aminoglycosides, antibacterial quinolones, antivirals, immune serum, immunoglobulins, antineoplastic agents, immunomodulatory agents, alkylation agents, antimetabolites, plant alkaloids and other natural products, cytotoxic antibiotics, immunosuppressive agents, drugs for disorders of the musculoskeletal system, antirheumatics, muscle relaxant agents, agents which affect bone structure and mineralization, drugs which act on the nervous system, general anesthetics, local anesthetics, opioids, antimigraine agents, anticonvulsants, anticholinergic agents, dopaminergic agents, antipsychotics, anxiolytics, hypnotics, sedatives, antidepressants, psychostimulants, anti-dementia drugs, parasympathomimetics, drugs used in addictive disorders, anti-vertigo agents, antiparasitic agents, insecticides, insect repellants, nasal decongestants, mucolytic agents, cough suppressants, ophthalmic active ingredients, otological active ingredients, antiglaucoma drugs, miotics, mydriatics, cycloplegics and/or mixtures thereof.

The delivery system of this invention can also be adsorbed on solid organic polymers or solid mineral supports, for example and not restricted to, talc, bentonite, silica, starch or maltodextrin among others.

In another particular embodiment, the delivery system of this invention which contains cosmetic and/or pharmaceutical active ingredients and/or adjuvants can be applied to the natural or synthetic fibers of textile materials before or after their manufacture. In this invention textile materials are understood to be woven fabrics, non-woven fabrics, garments and medical devices. These textile materials, in direct contact with the body's skin, release the active ingredients incorporated into the delivery system of this invention either by biodegradation of the of the binding system to the woven fabric, non-woven fabric or medical device or due to friction between these and the body, due to body moisture, the pH of the skin or body temperature. Examples of woven fabrics, non-woven fabrics, garments, medical devices and means for immobilization of delivery systems can be found in the prior art *("*Impregnating Fabrics With Microcapsules", HAPPI May 1986; Int. J. Pharm. 2002, 242, 55-62; *"*Biofunctional Textiles and the Skin" Curr. Probl. Dermatol. 2006 v.3; J. Cont. Release 2004, 97, 313-320). Means for immobilization of delivery systems in preferred textile materials are the application by means of a foulard, exhaustion bath or spraying. The natural and/or synthetic fibers can be wool, cotton, silk, nylon fibers, cellulose, polyamide or polyester among others. Among the textile materials the preferred woven fabrics, non-woven fabrics, garments and medical devices are bandages, gauzes, t-shirts, socks, tights, underwear, girdles, gloves, diapers, sanitary napkins, dressings, bedspreads, wipes, hydrogels, adhesive patches, non-adhesive patches, micro-electric patches and/or face masks.

According to another aspect, this invention relates to a cosmetic, pharmaceutical and/or alimentary composition which comprises the delivery system of this invention.

The delivery system of this invention can be incorporated into any form of functional alimentary or enriched alimentary, or into oral nutricosmetics or cosmetics, and formulated with the usual excipients and adjuvants for oral compositions or alimentary supplements, for example and not restricted to, common fatty components, aqueous components, humectants, preservatives, texturizing agents, flavors, aromas, antioxidants and colorants in the alimentary industry.

The cosmetic, pharmaceutical and/or alimentary compositions which comprise the delivery system of this invention can be prepared by the conventional methods known by the people skilled in the art *("*Harry's Cosmeticology", Eight edition 2000; *"*Remington: The Science and Practice of Pharmacy", Twentieth edition 2003*).* The cosmetic, pharmaceutical and/or alimentary compositions which incorporate the delivery system of this invention can be a final composition, available for application without having to carry out any kind of concentration, solution, dilution, dispersion, pulverization, spraying procedure or any other similar procedure known by the person skilled in the art, or an intermediate composition to which one or several of the previous procedures will be carried out or any other procedure known by the person skilled in the art with the aim of obtaining a final composition.

The cosmetic, pharmaceutical and/or alimentary compositions which comprise the delivery system of this invention can be administered by topical or transdermal administration, orally, or by any other type of suitable route, for example parenteral, for which the cosmetic and/or pharmaceutically acceptable excipients necessary for the formulation of the desired method of administration will be included. In the context of this invention, the term "parenteral" includes nasal, auricular, ophthalmic, rectal, urethral, vaginal routes, subcutaneous, intradermal, intravascular injections, such as intravenous, intramuscular, intraocular, intravitreous, intracorneal, intraspinal, intramedullary, intracranial, intracervical, intracerebral, intrameningeal, intraarticular, intrahepatic, intrathoracic, intratracheal, intrathecal and intraperitoneal, as well as any another similar injection or infusion technique. A review of the different pharmaceutical forms of administration of the active ingredients and excipients necessary for obtaining them can be found, for example, in the *"*Tratado de Farmacia Galénica", C. Faulí i Trillo, 1993, Luzán 5, S.A. Ediciones, Madrid*.*

The cosmetic and/or pharmaceutical compositions which comprise the delivery system of this invention can be used in different types of compositions of topical and transdermal application which optionally include the cosmetic and/or pharmaceutically acceptable excipients necessary for the formulation of the desired method of Administration *[*Faulí i Trillo C. (1993) en "Tratado de Farmacia Galénica", Luzán 5, S.A. Ediciones, Madrid*].* The compositions of topical or transdermal application may be presented in any solid, liquid or semi-solid formulation, for example and not restricted to, creams, multiple emulsions, for example and not restricted to, oil and/or silicone in water emulsions, water-in-oil and/or silicone emulsions, water/oil/water or water/silicone/water type emulsions, and oil/water/oil or silicone/water/silicone type emulsions, anhydrous compositions, aqueous dispersions, oils, milks, balsams, foams, lotions, gels, cream gels, hydroalcoholic solutions, hydroglycolic solutions, hydrogels, liniments, sera, soaps, shampoos, conditioners, serums, polysaccharide films, ointments, mousses, pomades, powders, bars, pencils and sprays or aerosols (sprays), including leave-on and rinse-off formulations. These formulations of topical and transdermal application can be incorporated by techniques known by the people skilled in the art into different types of solid accessories, for example and not restricted to, bandages, gauzes, t-shirts, socks, tights, underwear, girdles, gloves, diapers, sanitary napkins, dressings, bedspreads, wipes, adhesive patches, non-adhesive patches, microelectric patches or face masks, or can be incorporated into different make-up products such as make-up foundation, for example fluid foundations and compact foundations, make-up removal lotions, make-up removal milks, under-eye concealers, eye shadows, lipsticks, lip protectors, lip gloss and powders, among others. The cosmetic or dermopharmaceutical compositions of this invention can also be incorporated into products for the treatment and/or care of nails and cuticles such as nail varnishes, nail varnish remover lotions and cuticle remover lotions, among others.

The cosmetic, pharmaceutical and/or alimentary compositions which comprise the delivery system of this invention can be used in different types of formulations for oral administration, preferably in the form of oral cosmetics or drugs, for example and not restricted to, capsules, including gelatin capsules, soft capsules, hard capsules, tablets, including sugar coated tablets, powders, granules, chewing gum, solutions, suspensions, emulsions, syrups, polysaccharide films, jellies or gelatins, and any other form known by the person skilled in the art.

In another particular embodiment, the compositions which comprise the delivery system of this invention can be used for the treatment of textile materials and can be found in washing agents in liquid form, as well as detergents, in the manufacturing of emulsions, rinse aids, rinsing agents, fabric softener, sprays, liquid soaps or gels, or also in solid form, such as powder, granules or compact products. In addition, these compositions contain other components, for example and not restricted to, surfactants, agents which increase percutaneous absorption, agents for the prior treatment of textile materials, agents for the treatment of marks, abrasives, water softeners, fabric softeners, solvents or solubilizing agents, agents for the variation of touch and finish, dirt-repelling agents, antistatic agents, enzymes, agents which aid ironing, color and/or colorant brightening agents, shine agents, optical clearing agents, graying inhibitors or compounds for the loosening of dirt, color transfer inhibitors, phobizing and impregnating agents, swelling or thickening agents, consistency-generating agents, silicon agents, agents which increase the percutaneous absorption of microcapsules, whitening agents and textile material bleaching activators, hydrophilization agents and/or mixtures thereof.

Another aspect of this invention relates to the use of cosmetic, pharmaceutical and/or alimentary compositions which comprise the delivery system of this invention for the treatment and/or care of the skin, scalp, hair and nails. Preferably the treatment and/or care of the skin, hair, scalp and/or nails is selected from the group formed by treatment and/or prevention of skin aging, healing of the skin and/or scalp, dermatological treatment of skin diseases, treatment and/or prevention of cellulitis, tanning of the skin, lightening of the color or bleaching of the skin and treatment and/or prevention of hair loss.

In the context of this invention, the term "aging" relates to the changes experienced by the skin with age (chronoaging) or due to exposure to the sun (photoaging) or to environmental agents such as tobacco smoke, extreme climatic conditions of cold or wind, chemical pollutants or pollution, and includes all the external visible changes as well as those noticeable by touch, for example and not restricted to, the development of discontinuities on the skin such as wrinkles, fine lines, furrows, irregularities or roughness, increase in the size of pores, loss of elasticity, loss of firmness, loss of smoothness, loss of the capacity to recover from the deformation, sagging of the skin such as sagging cheeks, the appearance of bags under the eyes or the appearance of a double chin among others, changes to the color of the skin such as marks, reddening, bags under the eyes, appearance of hyperpigmented areas such as age spots or freckles among others, anomalous differentiation, hyperkeratinization, elastosis, keratosis, hair loss, orange-peel skin, loss of collagen structure and other histological changes of the stratum corneum, of the dermis, epidermis, vascular system (for example the appearance of spider veins or telangiectasias) or of tissues close to the skin, among others.

Another aspect of this invention relates to the use of cosmetic, pharmaceutical and/or alimentary compositions which comprise the delivery system of this invention for the treatment of textile materials. In this invention textile materials are understood to be woven fabrics, non-woven fabrics, garments and medical devices. Within textile materials the preferred woven fabrics, non-woven fabrics, garments and medical devices are bandages, gauzes, t-shirts, socks, tights, underwear, girdles, gloves, diapers, sanitary napkins, dressings, bedspreads, wipes, hydrogels, adhesive patches, non-adhesive patches, microelectric patches and/or face masks.

The following specific examples provided herein serve to illustrate the nature of this invention. These examples are included solely for illustrative purposes and should not be construed as limitations on the invention claimed herein.

### EXAMPLES

### General Methodology

All the reagents and solvents are of synthesis quality and are used without any additional treatment.

The high-pressure homogenizations were carried out in a "M110-Y" model microfluidizer by Microfluidics. The Ultraturrax mixer for the formation of microemulsions is the "D-8" model by Miccra RT.

### Example 1

### Preparation of lipid nanoparticle coacervate capsules: capsules with Octopirox^{®}.

Water, Zemea [INCI: PROPANEDIOL], phenoxyethanol [INCI: PHENOXYETHANOL] and sodium hyaluronate [INCI: SODIUM HYALURONATE] (ingredients A) were mixed together in a suitable vessel. Next Centrolex F [INCI: LECITHIN] (ingredient B) was slowly added under intense helix stirring until complete dispersion. Without ceasing the stirring, Inutec SP-1 [INCI: INULIN LAURYL CARBAMATE; WATER (AQUA); ETHYL PYRROLIDONE] (ingredient C) was added until complete homogeneity was achieved. At this point the resulting mixture of ingredients A, B and C was taken to a temperature of 65°C using a bain marie.

In another vessel ingredients D: MYRITOL 318 [INCI: CAPRYLIC/CAPRIC TRIGLYCERIDE], OCTOPIROX^{®} [INCI: PIROCTONE OLAMINE], CUTINA CP [INCI: CETYL PALMITATE], CUTINA CBS [INCI: GLYCERYL STEARATE; COCOGLYCERIDES; CETEARYL ALCOHOL; CETYL PALMITATE] and DERMOFEEL PS [INCI: POLYGLYCERYL-3 STEARATE] were mixed together and heated to 80°C, occasionally stirring until achieving homogeneity of the mixture, which was liquid at this temperature.

The D mixture was slowly added to the previous mixture of A+B+C under strong mechanical stirring, and once it had been added stirring was continued for 15 minutes to form a homogenous emulsion.

The sample was passed, without cooling, through a microfluidizer for three cycles at an entrance pressure of 80 bar and 15000 psi on exit, maintaining the operation temperature at between 65 and 75°C.

Solution E was added to the suspension of particles obtained, which was achieved by diluting Quat Soy LDMA 25 [INCI: WATER (AQUA); LAURYLDIMONIUM HYDROXYPROPYL HYDROLYZED SOY PROTEIN] in water under light stirring, to finally obtain a homogenous suspension of encapsulated lipid nanoparticles.

| | INGREDIENT (INCI Nomenclature) | % IN WEIGHT |
|---|---|---|
| A | WATER (AQUA) | QSP100 |
| A | PROPANEDIOL | 5.00 |
| A | PHENOXYETHANOL | 2.00 |
| A | SODIUM HYALURONATE | 0.01 |
| B | LECITHIN | 5.00 |
| C | INULIN LAURYL CARBAMATE; WATER (AQUA); ETHYL PYRROLIDONE | 1.00 |
| D | CAPRYLIC/CAPRIC TRIGLYCERIDE | 7.50 |
| D | PIROCTONE OLAMINE | 1.00 |
| D | CETYL PALMITATE | 3.00 |
| D | GLYCERYL STEARATE; COCOGLYCERIDES; CETEARYL ALCOHOL; CETYL PALMITATE | 3.00 |
| D | POLYGLYCERYL-3 STEARATE | 1.00 |
| E | WATER (AQUA); LAURYLDIMONIUM HYDROXYPROPYL HYDROLYZED SOY PROTEIN | 0.20 |
| E | WATER (AQUA) | 2.00 |

The average size of the capsules in suspension obtained determined by Dynamic Laser Light Scattering was 198 nm.

The efficacy of encapsulation was determined by passing an aliquot of the suspension of nanoparticles through a Sephadex 50 column, centrifuging and subsequently determining the concentration of the active ingredient Octopirox [INCI: PIROCTONE OLAMINE] obtained in the different separated fractions. This determination was carried out by the method supplied by the manufacturer of Octopirox^{®} consisting of the spectrophotometric quantification of a piroctone olamine complex with Fe³⁺ [*Colorimetric determination of Octopirox in ready-to-use cosmetic formulation, operating procedure* EEH-1200-AA-0036, version 1, 2002, by Clariant GMBH]. The efficacy of encapsulation was 87.1%.

### Example 2

### Preparation of lipid nanoparticle coacervation capsules: capsules witch Lipochroman-6.

Inutec SP-1 [INCI: INULIN LAURYL CARBAMATE] was dissolved in water in a suitable vessel. Next, Centrolex F [INCI: LECITHIN] (ingredient A) was slowly added and the mixture was heated to 60-70°C.

In another vessel MYRITOL 318 [INCI: CAPRYLIC/CAPRIC TRIGLYCERIDE], Lipochroman-6 [INCI: DIMETHYLMETHOXY CHROMANOL], Cutina CP [INCI: CETYL PALMITATE], Cutina CR [INCI: CETYL RICINOLEATE] and DERMOFEEL PS [INCI: POLYGLYCERYL-3 STEARATE] (ingredients B) were mixed together. The mixture was heated to 80-90°C in a water bath until totally dissolved.

Next, ingredients B were slowly added to ingredients A under intense stirring until achieving a suitable emulsion and the mixture was left being stirring until it reached room temperature.

In another vessel hyaluronic acid [INCI: SODIUM HYALURONATE] was dissolved in water (ingredient C). Once dissolved it was added to the previously prepared emulsion.

Next, Quat Soy LDMA 25 [INCI: WATER (AQUA); LAURYLDIMONIUM HYDROXYPROPYL HYDROLYZED SOY PROTEIN] was added dissolved in water under stirring (ingredients D).

| | INGREDIENT (INCI Nomenclature) | % IN WEIGHT |
|---|---|---|
| A | WATER (AQUA) | QSP100 |
| A | INULIN LAURYL CARBAMATE | 1.00 |
| A | LECITHIN | 5.00 |
| B | CAPRYLIC/CAPRIC TRIGLYCERIDE | 5.00 |
| B | DIMETHYLMETHOXY CHROMANOL | 1.00 |
| B | CETYL PALMITATE | 3.00 |
| B | CETYL RICINOLEATE | 1.00 |
| B | POLYGLYCERYL-3 STEARATE | 3.00 |
| C | SODIUM HYALURONATE | 0.01 |
| D | LAURYLDIMONIUM HYDROXYPROPYL HYDROLYZED SOY PROTEIN | 0.20 |
| D | WATER (AQUA) | 2.00 |

### Example 3

### Preparation of lipid nanoparticle coacervate capsules: capsules with retinol and Lipochroman-6.

In a suitable vessel water, Inutec SP-1 [INCI: INULIN LAURYL CARBAMATE], Zemea [INCI: PROPANEDIOL] and phenoxyethanol [INCI: PHENOXYETHANOL] (ingredients A) were added in this order.

To the mixture of ingredients A Centrolex F [INCI: LECITHIN] (ingredient B) was added drop by drop under intense stirring.

In another vessel soybean oil [INCI: GLYCINE SOJA (SOYBEAN) OIL], Lipochroman-6 [INCI: DIMETHYLMETHOXY CHROMANOL], Cutina CP [INCI: CETYL PALMITATE], Cutina CR [INCI: CETYL RICINOLEATE], Cutina CBS [INCI: COCOGLYCERIDES] and Dermofeel PS [INCI: POLYGLYCERYL-3 STEARATE] (ingredients C) were mixed together. The mixture was heated until all the ingredients merged together.

Next, Retinol S10 [INCI: RETINOL] (ingredient D) was added to the mixture of ingredients C.

Maintaining phases A+B and C+D at 80°C, C+D was slowly added to A+B under intense stirring until an emulsion was formed. The heated mixture was stirred vigorously with Ultraturrax at 5000 rpm for 30 minutes. Once emulsified, the pH was checked and adjusted to 5.50; the mixture was allowed to cool to room temperature whilst being stirred with a turbine.

Next, a suspension in water of Quat Soy LDMA 25 [INCI: LAURYLDIMONIUM HYDROXYPROPYL HYDROLYZED SOY PROTEIN] (ingredients E) was added drop by drop under stirring. Lastly, hyaluronic acid was added [INCI: SODIUM HYALURONATE] and stirring was maintained for 30 minutes until a suitable emulsion was obtained.

| | INGREDIENT (INCI Nomenclature) | % IN WEIGHT |
|---|---|---|
| A | WATER (AQUA) | QSP100 |
| A | INULIN LAURYL CARBAMAT | 5.00 |
| A | PROPANEDIOL | 5.00 |
| A | PHENOXYETHANOL | 2.60 |
| B | LECITHIN | 5.00 |
| C | GLYCINE SOJA (SOYBEAN) OIL | 5.00 |
| C | DIMETHYLMETHOXY CHROMANOL | 0.10 |
| C | CETYL PALMITATE | 3.00 |
| C | CETYL RICINOLEATE | 1.00 |
| C | COCOGLYCERIDES | 1.00 |
| C | POLYGLYCERYL-3 STEARATE | 3.00 |
| D | RETINOL | 2.00 |
| E | WATER (AQUA) | 2.00 |
| E | LAURYLDIMONIUM HYDROXYPROPYL HYDROLYZED SOY PROTEIN | 0.20 |
| F | WATER (AQUA) | 10.00 |
| F | SODIUM HYALURONATE | 0.01 |

### Example 4

### Preparation of lipid nanoparticle coacervate capsules: capsules with coenzyme Q10 and Lipochroman-6.

In a suitable vessel water, Structure XL [INCI: HYDROXYPROPYL STARCH PHOSPHATE], Amigel [INCI: SCLEROTIUM GUM], Hyaluronic acid [INCI: SODIUM HYALURONATE], Zemea [INCI: PROPANEDIOL] and phenoxyethanol [INCI: PHENOXYETHANOL] (ingredients A) were added in this order. The mixture was heated in a microwave to 60-65°C.

In another vessel Lipochroman-6 [INCI: DIMETHYLMETHOXY CHROMANOL], Coenzyme Q10 [INCI: UBIQUINONE], Myritol 318 [INCI: CAPRYLIC/CAPRIC TRIGLYCERIDE], Lanette O [INCI: CETEARYL ALCOHOL], Emulgade SE DF [INCI: CETEARETH-12] and Arlamol HD [INCI: ISOHEXADECANE] (ingredients B) were mixed together. The mixture was heated to 80-85°C until the ingredients merged together.

The mixture of ingredients B was added to the mixture of ingredients A, whilst being stirred with a turbine until a good emulsion was obtained.

Next, a suspension in water of Quat Soy LDMA 25 [INCI: LAURYLDIMONIUM HYDROXYPROPYL HYDROLYZED SOY PROTEIN] (ingredients C) was added drop by drop under stirring; the stirring was maintained for 10 minutes.

Finally, the mixture was homogenized under pressure in a microfluidizer for 3 cycles with an entrance pressure of 80 bar and pressure on exit of 15000 psi. Next, the homogenized mixture was allowed to cool to room temperature whilst being stirred with a turbine.

| | INGREDIENT (INCI Nomenclature) | % IN WEIGHT |
|---|---|---|
| A | WATER (AQUA) | QSP100 |
| A | HYDROXYPROPYL STARCH PHOSPHATE | 1.00 |
| A | SCLEROTIUM GUM | 0.50 |
| A | SODIUM HYALURONATE | 0.01 |
| A | PROPANEDIOL | 5.00 |
| A | PHENOXYETHANOL | 2.60 |
| B | DIMETHYLMETHOXY CHROMANOL | 0.10 |
| B | UBIQUINONE | 5.00 |
| B | CAPRYLIC/CAPRIC TRIGLYCERIDE | 5.00 |
| B | CETEARYL ALCOHOL | 2.00 |
| B | CETEARETH-12 | 3.50 |
| B | ISOHEXADECANE | 1.00 |
| C | WATER (AQUA) | 2.00 |
| C | LAURYLDIMONIUM HYDROXYPROPYL HYDROLYZED SOY PROTEIN | 0.20 |

The average size of the capsules in suspension obtained determined by Dynamic Laser Light Scattering was 183 nm.

### Example 5

### Preparation of lipid nanoparticle coacervate capsules: capsules with microemulsified vitamin C.

### 0.1% Vitamin C microemulsion

In a suitable vessel Ducosate [INCI: DIETHYLHEXYL SODIUM SULFOSUCCINATE] and isostearic acid [INCI: ISOSTEARIC ACID] were mixed together (phase A).

In another vessel the vitamin C [INCI: ASCORBIC ACID] was dissolved in water. Once dissolved, the ethanol [INCI: ALCOHOL] was added (phase B).

Phase B was slowly added to phase A under stirring.

| | INGREDIENT (INCI Nomenclature) | % IN WEIGHT |
|---|---|---|
| A | DIETHYLHEXYL SODIUM SULFOSUCCINATE/ ISOSTEARIC ACID (15/85) | 89.90 |
| B | ASCORBIC ACID | 0.10 |
| B | WATER (AQUA) | 5.00 |
| B | ALCOHOL | 5.00 |

### Coacervate capsules with lipid nanoparticles and microemulsified vitamin C

In a suitable vessel water, Amigel [INCI: SCLEROTIUM GUM], Zemea [INCI: PROPANEDIOL] and phenoxyethanol [INCI: PHENOXYETHANOL] (ingredients A) were mixed together and the mixture was heated in a microwave to approximately 50°C.

In another vessel, the 0.1% microemulsion of vitamin C, soybean oil [INCI: GLYCINE SOJA (SOYBEAN) OIL], Lanette O [INCI: CETEARYL ALCOHOL], Emulgade SE DF [INCI: CETEARETH-12] and Arlamol HD [INCI: ISOHEXADECANE] (ingredients B) were mixed together. The mixture was heated until all the ingredients were merged together.

Next, the mixture of ingredients B was added to the mixture of ingredients A, under stirring over about 10 minutes. The hot mixture was stirred with Ultraturrax at 5000 rpm for 30 minutes. Once closely emulsified, the pH was checked and adjusted to 5.50. The mixture was allowed to cool to room temperature whilst being stirred with a turbine.

The hyaluronic acid was added [INCI: SODIUM HYALURONATE] (ingredient C) to the mixture of A+B under stirring until it was well homogenized.

Finally, a suspension in water of Quat Soy LDMA 25 [INCI: LAURYLDIMONIUM HYDROXYPROPYL HYDROLYZED SOY PROTEIN] (ingredients D) was added drop by drop under stirring, and the mixture was allowed to cool whilst being stirred with a turbine.

| | INGREDIENT (INCI Nomenclature) | % IN WEIGHT |
|---|---|---|
| A | WATER (AQUA) | QSP100 |
| A | SCLEROTIUM GUM | 0.50 |
| A | PROPANEDIOL | 5.00 |
| A | PHENOXYETHANOL | 2.6 |
| B | DIETHYLHEXYL SODIUM SULFOSUCCINATE/ ISOSTEARIC ACID (15/85), ASCORBIC ACID, WATER (AQUA), ALCOHOL | 10.00 |
| B | GLYCINE SOJA (SOYBEAN) OIL | 10.00 |
| B | CETEARYL ALCOHOL | 2.00 |
| B | CETEARETH-12 | 3.50 |
| B | ISOHEXADECANE | 1.00 |
| C | SODIUM HYALURONATE | 0.01 |
| D | WATER (AQUA) | 2.00 |
| D | LAURYLDIMONIUM HYDROXYPROPYL HYDROLYZED SOY PROTEIN | 0.20 |

The average size of the capsules in suspension obtained determined by Dynamic Laser Light Scattering was 155 nm.

### Example 6

### Preparation of hydrosoluble peptide microemulsions for their subsequent encapsulation in coacervate capsules which contain lipid nanoparticles.

### Example 6-a. Microemulsion of Inyline^{™}

In a suitable vessel Ducosate [INCI: DIETHYLHEXYL SODIUM SULFOSUCCINATE] and isostearic acid [INCI: ISOSTEARIC ACID] were mixed together (phase A).

In another vessel the peptide Inyline^{™} [INCI: ACETYL HEXAPEPTIDE-30] was dissolved in ethanol [INCI: ALCOHOL]. Once dissolved, it was added to the water (phase B).

Phase B was slowly added to phase A under stirring.

| | INGREDIENT (INCI Nomenclature) | % IN WEIGHT |
|---|---|---|
| A | DIETHYLHEXYL SODIUM SULFOSUCCINATE/ ISOSTEARIC ACID (15/85) | 89.955 |
| B | ACETYL HEXAPEPTIDE-30 | 0.045 |
| B | WATER (AQUA) | 2.00 |
| B | ALCOHOL | 8.00 |

### Example 6-b. Microemulsion of Argireline^{®}

In a suitable vessel Ducosate [INCI: DIETHYLHEXYL SODIUM SULFOSUCCINATE] and isostearic acid [INCI: ISOSTEARIC ACID] were mixed together (phase A).

In another vessel the peptide Argilerine^{®} [INCI: ACETYL HEXAPEPTIDE-8] was dissolved in water. Once dissolved, ethanol [INCI: ALCOHOL] was added (phase B). Phase B was slowly added to phase A under stirring.

| | INGREDIENT (INCI Nomenclature) | % IN WEIGHT |
|---|---|---|
| A | DIETHYLHEXYL SODIUM SULFOSUCCINATE/ ISOSTEARIC ACID (15/85) | 89.75 |
| B | ACETYL HEXAPEPTIDE-8 | 0.25 |
| B | WATER (AQUA) | 5.00 |
| B | ALCOHOL | 5.00 |

### Example 6-c. Microemulsion of Eyeseryl^{®}

In a suitable vessel Ducosate [INCI: DIETHYLHEXYL SODIUM SULFOSUCCINATE] and isostearic acid [INCI: ISOSTEARIC ACID] were mixed together (phase A).

In another vessel the peptide Eyeseryl^{®} [INCI: ACETYL TETRAPEPTIDE-5] was dissolved in water. Once dissolved, ethanol [INCI: ALCOHOL] was added (phase B). Phase B was slowly added to phase A under stirring.

| | INGREDIENT (INCI Nomenclature) | % IN WEIGHT |
|---|---|---|
| A | DIETHYLHEXYL SODIUM SULFOSUCCINATE/ ISOSTEARIC ACID (15/85) | 89.50 |
| B | ACETYL TETRAPEPTIDE-5 | 0.50 |
| B | WATER (AQUA) | 5.00 |
| B | ALCOHOL | 5.00 |

### Example 6-d. Microemulsion of Decorinol

In a suitable vessel Ducosate [INCI: DIETHYLHEXYL SODIUM SULFOSUCCINATE] and isostearic acid [INCI: ISOSTEARIC ACID] were mixed together (phase A).

In another vessel the peptide Decorinol [INCI: TRIPEPTIDE-9 CITRULLINE] was dissolved in water. Once dissolved, ethanol [INCI: ALCOHOL] was added (phase B). Phase B was slowly added to phase A under stirring.

| | INGREDIENT (INCI Nomenclature) | % IN WEIGHT |
|---|---|---|
| A | DIETHYLHEXYL SODIUM SULFOSUCCINATE/ ISOSTEARIC ACID (15/85) | 89.75 |
| B | TRIPEPTIDE-9 CITRULLINE | 0.25 |
| B | WATER (AQUA) | 5.00 |
| B | ALCOHOL | 5.00 |

### Example 6-e. Microemulsion of Decorinyl^{®}

In a suitable vessel Ducosate [INCI: DIETHYLHEXYL SODIUM SULFOSUCCINATE] and isostearic acid [INCI: ISOSTEARIC ACID] were mixed together (phase A).

In another vessel the peptide Decorinyl^{®} [INCI: TRIPEPTIDE-10 CITRULLINE] was dissolved in water. Once dissolved, ethanol [INCI: ALCOHOL] was added (phase B). Phase B was slowly added to phase A under stirring.

| | INGREDIENT (INCI Nomenclature) | % IN WEIGHT |
|---|---|---|
| A | DIETHYLHEXYL SODIUM SULFOSUCCINATE/ ISOSTEARIC ACID (15/85) | 89.75 |
| B | TRIPEPTIDE-10 CITRULLINE | 0.25 |
| B | WATER (AQUA) | 5.00 |
| B | ALCOHOL | 5.00 |

### Example 6-f. Microemulsion of SNAP-7

In a suitable vessel Ducosate [INCI: DIETHYLHEXYL SODIUM SULFOSUCCINATE] and isostearic acid [INCI: ISOSTEARIC ACID] were mixed together (phase A).

In another vessel the peptide SNAP-7 [INCI: ACETYL HEPTAPEPTIDE-4] was dissolved in water. Once dissolved, ethanol [INCI: ALCOHOL] was added (phase B). Phase B was slowly added to phase A under stirring.

| | INGREDIENT (INCI Nomenclature) | % IN WEIGHT |
|---|---|---|
| A | DIETHYLHEXYL SODIUM SULFOSUCCINATE/ ISOSTEARIC ACID (15/85) | 89.75 |
| B | ACETYL HEPTAPEPTIDE-4 | 0.25 |
| B | WATER (AQUA) | 5.00 |
| B | ALCOHOL | 5.00 |

### Example 6-g. Microemulsion of SNAP-8

In a suitable vessel Ducosate [INCI: DIETHYLHEXYL SODIUM SULFOSUCCINATE] and isostearic acid [INCI: ISOSTEARIC ACID] were mixed together (phase A).

In another vessel the peptide SNAP-7 [INCI: ACETYL OCTAPEPTIDE-3] was dissolved in water. Once dissolved, ethanol [INCI: ALCOHOL] was added (phase B).

Phase B was slowly added to phase A under stirring.

| | INGREDIENT (INCI Nomenclature) | % IN WEIGHT |
|---|---|---|
| A | DIETHYLHEXYL SODIUM SULFOSUCCINATE/ ISOSTEARIC ACID (15/85) | 89.75 |
| B | ACETYL OCTAPEPTIDE-3 | 0.25 |
| B | WATER (AQUA) | 5.00 |
| B | ALCOHOL | 5.00 |

### Example 7

### Preparation of coacervate capsules of lipid nanoparticles: capsules with microemulsified hydrosoluble peptides.

In a suitable vessel water, Amigel [INCI: SCLEROTIUM GUM], hyaluronic acid [INCI: SODIUM HYALURONATE], Zemea [INCI: PROPANEDIOL] and phenoxyethanol [INCI: PHENOXYETHANOL] (ingredients A) were added in this order, and the mixture was heated in a microwave to approximately 80°C.

In another vessel, the microemulsion of the corresponding peptide prepared according to example 6, soybean oil [INCI: GLYCINE SOJA (SOYBEAN) OIL], Lanette O [INCI: CETEARYL ALCOHOL], Emulgade SE DF [INCI: CETEARETH-12] and Arlamol HD [INCI: ISOHEXADECANE] (ingredients B) were added. The mixture was heated to 80-85°C until all the ingredients had merged.

Next, the mixture of ingredients B was added to the mixture of ingredients A, whilst being stirred with a turbine until an emulsion was formed.

Next, a suspension in water of Quat Soy LDMA 25 [INCI: LAURYLDIMONIUM HYDROXYPROPYL HYDROLYZED SOY PROTEIN] (ingredients C) was added drop by drop under stirring.

Finally, the mixture was homogenized under pressure in a microfluidizer for 3 cycles with an entrance pressure of 80 bar and an exit pressure of 15000 psi. Next, the homogenized mixture was allowed to cool to room temperature whilst being stirred with a turbine.

For the peptide Inyline^{™} [INCI: ACETYL HEXAPEPTIDE-30], the microemulsion prepared according to *example 6-a* was used.

| | INGREDIENT (INCI Nomenclature) | % IN WEIGHT |
|---|---|---|
| A | WATER (AQUA) | QSP100 |
| A | SCLEROTIUM GUM | 0.50 |
| A | PROPANEDIOL | 5.00 |
| A | PHENOXYETHANOL | 2.6 |
| A | SODIUM HYALURONATE | 0.01 |
| B | ACETYL HEXAPEPTIDE-30, DIETHYLHEXYL SODIUM SULFOSUCCINATE/ ISOSTEARIC ACID (15/85), WATER (AQUA), ALCOHOL | 10.00 |
| B | GLYCINE SOJA (SOYBEAN) OIL | 10.00 |
| B | CETEARYL ALCOHOL | 2.00 |
| B | CETEARETH-12 | 3.50 |
| B | ISOHEXADECANE | 1.00 |
| C | WATER (AQUA) | 2.00 |
| C | LAURYLDIMONIUM HYDROXYPROPYL HYDROLYZED SOY PROTEIN | 0.20 |

The average size of the capsules in suspension with Inyline^{™} [INCI: ACETYL HEXAPEPTIDE-30] obtained determined by Dynamic Laser Light Scattering was 102 nm.

For the peptide Argilerine^{®} [INCI: ACETYL HEXAPEPTIDE-8], the microemulsion prepared according to *example 6-b* was used.

| | INGREDIENT (INCI Nomenclature) | % IN WEIGHT |
|---|---|---|
| A | WATER (AQUA) | QSP100 |
| A | SCLEROTIUM GUM | 0.50 |
| A | PROPANEDIOL | 5.00 |
| A | PHENOXYETHANOL | 2.6 |
| A | SODIUM HYALURONATE | 0.01 |
| B | ACETYL HEXAPEPTIDE-8, DIETHYLHEXYL SODIUM SULFOSUCCINATE/ ISOSTEARIC ACID (15/85), WATER (AQUA), ALCOHOL | 10.00 |
| B | GLYCINE SOJA (SOYBEAN) OIL | 10.00 |
| B | CETEARYL ALCOHOL | 2.00 |
| B | CETEARETH-12 | 3.50 |
| B | ISOHEXADECANE | 1.00 |
| C | WATER (AQUA) | 2.00 |
| C | LAURYLDIMONIUM HYDROXYPROPYL HYDROLYZED SOY PROTEIN | 0.20 |

The average size of the capsules in suspension with Argilerine^{®} [INCI: ACETYL HEXAPEPTIDE-8] obtained determined by Dynamic Laser Light Scattering was 104 nm.

For the peptide Eyeseryl^{®} [INCI: ACETYL TETRAPEPTIDE-5], the microemulsion prepared according to *example 6-c* was used.

| | INGREDIENT (INCI Nomenclature) | % IN WEIGHT |
|---|---|---|
| A | WATER (AQUA) | QSP100 |
| A | SCLEROTIUM GUM | 0.50 |
| A | PROPANEDIOL | 5.00 |
| A | PHENOXYETHANOL | 2.6 |
| A | SODIUM HYALURONATE | 0.01 |
| B | ACETYL TETRAPEPTIDE-5, DIETHYLHEXYL SODIUM SULFOSUCCINATE/ ISOSTEARIC ACID (15/85), WATER (AQUA), ALCOHOL | 20.00 |
| B | GLYCINE SOJA (SOYBEAN) OIL | 10.00 |
| B | CETEARYL ALCOHOL | 2.00 |
| B | CETEARETH-12 | 3.50 |
| B | ISOHEXADECANE | 1.00 |
| C | WATER (AQUA) | 2.00 |
| C | LAURYLDIMONIUM HYDROXYPROPYL HYDROLYZED SOY PROTEIN | 0.20 |

The average size of the capsules in suspension with Eyeseryl^{®} [INCI: ACETYL TETRAPEPTIDE-5] obtained determined by Dynamic Laser Light Scattering was 110 nm.

In the peptide encapsulations, the separation of the encapsulated and non-encapsulated active ingredient was carried out by the basket centrifugation technique [David W. Fry et al. Analytical Biochemistry 90: 809-815 (1978)]. Once both fractions had been separated, the non-encapsulated part was analyzed by HPLC. In no case was peptide presence detected in the aqueous phase of the dispersion, therefore, the efficacy of encapsulation is about 100%.

### Example 8

### Study of the comparative stability of Lipochroman-6 in different delivery systems.

When solutions of Lipochroman-6 [INCI: DIMETHYLMETHOXY CHROMANOL] come into contact with alcohols they present a change in coloring, changing from the original solution's transparent color to an intense red color over time.

To demonstrate the greater stabilization abilities of active ingredients of the lipid nanoparticles capsules, different delivery systems were prepared containing 0*.*1% Lipochroman-6 and were subjected to incubation at 40°C in the presence of alcohols for 48 hours.

### Preparation of different delivery systems assayed

### Example 8-a: Standard.

Solution of 0*.*1% Lipochroman-6 in ethanol (standard).

### Example 8-b: Preparation of a suspension of liposomes containing 1% Lipochroman-6.

In a suitable vessel water, vegetable ceramides [INCI: LECITHIN, GLYCOLIPIDS], a homogeneous dispersion of Lipochroman-6 [INCI: DIMETHYLMETHOXY CHROMAN] in PARSOL MCX [INCI: ETHYLHEXYL METHOXYCINNAMATE], a homogeneous mixture of ZEMEA [INCI: PROPANEDIOL] with phenoxyethanol [INCI: PHENOXYETHANOL], EMULMETIK 930 [INCI: LECITHIN], LECIFLOR 100 IP [INCI: Lecithin] were added in this order and under constant mechanical stirring. The mixture was heated to 60°C and INUTEC SP-1 [INCI: INULIN LAURYL CARBAMATE] was added.

The sample was passed through a microfluidizer without being cooled for three cycles at an entrance pressure of 80 bar and 15000 psi on exit.

| | INGREDIENT (INCI Nomenclature) | % IN WEIGHT |
|---|---|---|
| A | WATER (AQUA) | QSP100 |
| A | LECITHIN, GLYCOLIPIDS | 1.00 |
| B | DIMETHYLMETHOXY CHROMAN | 1.00 |
| B | ETHYLHEXYL METHOXYCINNAMATE | 7.00 |
| C | PROPANEDIOL | 20.00 |
| C | PHENOXYETHANOL | 2.60 |
| D | LECITHIN | 1.00 |
| E | LECITHIN | 3.00 |
| F | INULIN LAURYL CARBAMATE | 0.20 |

### Example 8-c: Preparation of a suspension of microparticles containing 1% Lipochroman-6.

The process was exactly the same as in example 2 without including phases C and D and carrying out the homogenization under light stirring.

In a suitable vessel water and Inutec SP-1 [INCI: INULIN LAURYL CARBAMATE] were added in this order. The mixture was stirred until Inutec SP-1 [INCI: INULIN LAURYL CARBAMATE] was correctly dissolved. Next Centrolex F [INCI: LECITHIN] (ingredients A) was slowly added and the mixture was heated to 60-70°C.

In another vessel MYRITOL 318 [INCI: CAPRYLIC/CAPRIC TRIGLYCERIDE], Lipochroman-6 [INCI: DIMETHYLMETHOXY CHROMANOL], Cutina CP [INCI: CETYL PALMITATE], Cutina CR [INCI: CETYL RICINOLEATE], DERMOFEEL PS [INCI: POLYGLYCERYL-3 STEARATE] (ingredients B) were mixed together. The mixture was heated to 80-90°C in a water bath until all the ingredients had completely dissolved.

The mixture of ingredients B was slowly added to the mixtures of ingredients A under light mechanical stirring until a suitable emulsion of observable size under an optical microscope was obtained (in the region of 2 µm). The final mixture was stirred until the temperature reached 25°C.

### Example 8-d: Preparation of a suspension of NLC containing Lipochroman-6.

The process was exactly the same as in example 2 without including phases C and D.

### Example 8-e: Suspension of capsules of lipid nanoparticles containing Lipochroman-6.

They were prepared in the same way as in example 2.

### Stability assays

The quantitative data from the coloration caused by the degradation of Lipochroman-6 was obtained by the UV/Visible Spectrophotometer technique, measuring the absorbency of the samples at 295 nm, all diluted at the same concentration.

The samples for the assay, except the standard, were prepared according to 1g solution of suspension of examples 8-*a* to 8-*d* in 10 ml of 10% aqueous solution of ethanol. Next, the samples were subjected to incubation at 40°C for 48 hours. Finally, the absorbency of the samples at 295 nm was measured after carrying out a 1/100 dilution in isopropanol.

The values obtained are shown in the table below:

| **SAMPLE** | **Absorbency (λ= 295 nm)** |
|---|---|
| *8-a* | 2.405 |
| *8-b* | 3.204 |
| *8-c* | 0.302 |
| *8-d* | 0.291 |
| *8-e* | 0.267 |

It was observed that the standard solution (sample *8-a*) and the suspension of liposomes (sample *8-b*) saturated the spectrophotometer signal, indicating their degradation in an alcoholic medium.

The lipid nanoparticles (sample *8-d*) provided a signal intensity lower than the standard solution (sample *8-a*), the liposomes (sample *8-b*), or the microparticles, but greater than that of the encapsulated lipid nanoparticles (sample *8-e*). Therefore, the protection of the active ingredient (Lipochroman-6) from chemical degradation was greater when it was in encapsulated lipid nanoparticles.

### Example 9

### Study of comparative stability of retinol in different delivery systems.

Retinol [INCI: DIMETHYLMETHOXY CHROMANOL] is a photolabile active ingredient. To demonstrate the greater stabilization ability of active ingredients of lipid nanoparticle capsules, different delivery systems containing 1% retinol were prepared and were subjected to incubation at 40°C for 3 months. The concentration of the active ingredient was determined by HPLC.

### Preparation of the different delivery systems assayed

### Example 9-a: Preparation of a 1% retinol emulsion.

It is prepared in exactly the same way as that described in example 3 substituting phase C for the following:
10% Soybean oil [INCI: GLYCINE SOJA (SOYBEAN) OIL]
0.1% Lipochroman-6 [INCI: DIMETHYLMETHOXY CHROMANOL].
Phases E and F of the preparation are also eliminated

### Example 9-b: Preparation of a suspension of coacervate nanocapsules containing retinol.

The coacervate nanocapsules were prepared in exactly the same way as that described in example 3 substituting phase C for the following:
10% Soybean oil [INCI: GLYCINE SOJA (SOYBEAN) OIL]
0.1% Lipochroman-6 [INCI: DIMETHYLMETHOXY CHROMANOL].

### Example 9-c: Preparation of a suspension of lipid nanoparticle capsules with retinol.

The preparation is described in example 3.

### Retinol stability assay

The emulsion samples *(9*-*a)*, nanocapsules *(9-b)* and lipid nanoparticle capsules *(9-c)* were incubated for 3 months at 40°C.

For the analysis, 1g of sample was taken and was diluted to 10 mL with isopropanol. The dilution mixture was subjected to ultrasonication for 5 minutes and was filtered using paper to eliminate the wax particles. Finally a 1/25 dilution in isopropanol was carried out, and the simple was analyzed by HPLC with a Nucleosil C18 column, using a methanol/water gradient and measuring the absorbency signal at 326 nm.

The results obtained were those shown in the table below:

| **SAMPLE** | **Retinol (%)** | |
|---|---|---|
| | **Initial** | **3 months** |
| *9-a* | 1.0 | 0.29 |
| *9-b* | 1.0 | 0.41 |
| *9-c* | 1.0 | 0.73 |

It could be observed that the lipid nanoparticle capsule system would grant retinol more stability than the nanoparticles obtained by complex coacervation, and much more than the simple retinol emulsion.

### Example 10

### Comparative percutaneous absorption test of different delivery systems.

Percutaneous absorption is a process through which a certain active ingredient passes through the different layers of skin. This process can be divided into 3 principal stages: penetration, permeation, permeation and resorption. Penetration is the entrance of the active ingredient into a certain layer of skin. Permeation is passing through one layer of skin to another structurally different layer. And resorption is the entrance of active ingredient into the vascular system.

### Preparation of the different delivery systems assayed

### Example 10-a: Standard

2% solution of caffeine in water.

### Example 10-b: Preparation of a suspension of multilamellar liposomes with caffeine.

In a suitable vessel water, disodium EDTA [INCI: DISODIUM EDTA], Phenonip [INCI: PHENOXYETHANOL, METHYLPARABEN, ETHYLPARABEN, BUTYLPARABEN, PROPYLPARABEN, ISOBUTYLPARABEN] and Abiol [INCI: IMIDAZOLIDINYL UREA] (ingredients A) were added in this order, and the mixture was mechanically stirred until a homogeneous dispersion was obtained. The pH fell to 3 with a solution of citric acid (ingredient B).

Without ceasing the stirring, caffeine [INCI: CAFFEINE] (ingredient C), CENTROLEX F [INCI: LECITHIN] (ingredient D) were added and stirring was continued for an extra 30 minutes. Subsequently, the ingredients E: TEAL [INCI: CARBOMER] and carraghenates [INCI: CARRAGEENAN (CHONDRUS CRISPUS)] were added, and the pH was adjusted to 6.3 with triethanolamine (ingredient F).

| | INGREDIENT (INCI Nomenclature) | % IN WEIGHT |
|---|---|---|
| A | WATER (AQUA) | QSP100 |
| A | DISODIUM EDTA | 0.15 |
| A | PHENOXYETHANOL, METHYLPARABEN, ETHYLPARABEN,BUTYLPARABEN, PROPYLPARABEN, ISOBUTYLPARABEN | 0.38 |
| A | IMIDAZOLIDINYL UREA | 0.10 |
| B | CITRIC ACID, WATER (AQUA) | q.s. |
| C | CAFFEINE | 2.00 |
| D | LECITHIN | 4.00 |
| E | CARBOMER | 3.50 |
| E | CARRAGEENAN (CHONDRUS CRISPUS) | 1.00 |
| F | TRIETHANOLAMINE | q.s. |

### Example 10-c: Preparation of a suspension of microfluidified liposomes with caffeine.

They are prepared in exactly the same way as in the previous example incorporating a microfluidification step, with 2 cycles at 15000 psi on exit and 80 bar entrance after step D and before step E.

### Example 10-d: Preparation of a suspension of mixed micelles with caffeine.

In a suitable vessel water adjusted to pH 3 with citric acid [INCI: CITRIC ACID], caffeine [INCI: CAFFEINE], Dermosoft Octiol [INCI: CAPRYLYL GLYCOL], Sensiva SC [INCI: ETHYLHEXYLGLYCERIN] and phenoxyethanol [INCI: PHENOXYETHANOL] (ingredients A) were added in this order, and the mixture was mechanically stirred at 50°C until a homogeneous dispersion was obtained. The pH fell to 3 with a solution of citric acid (ingredient B)

Without ceasing the stirring, CENTROLEX F [INCI: LECITHIN] (ingredient B), and Oramix [INCI: CAPRYLYL/CAPRYL GLUCOSIDE, WATER (AQUA)] (ingredient E) were added. The final mixture was homogenized under high pressure in a microfluidizer for 2 cycles at a pressure of 15000 psi on exit and 80 bar on entrance.

| | INGREDIENT (INCI Nomenclature) | % IN WEIGHT |
|---|---|---|
| A | WATER (AQUA), CITRIC ACID | QSP100 |
| A | CAFFEINE | 2.0 |
| A | CAPRYLYL GLYCOL | 0.5 |
| A | ETHYLHEXYLGLYCERIN | 0.5 |
| A | PHENOXYETHANOL | 0.7 |
| B | LECITHIN | 3.0 |
| E | CAPRYLYL/CAPRYL GLUCOSIDE WATER (AQUA) | 30.0 |

### Example 10-e: Preparation of a 0.5% microemulsion with caffeine.

In a suitable vessel Ducosate [INCI: DIETHYLHEXYL SODIUM SULFOSUCCINATE] and isostearic acid [INCI: ISOSTEARIC ACID] were mixed together (phase A).

In another vessel caffeine [INCI: CAFFEINE] was dissolved in water. Once dissolved, ethanol was added [INCI: ALCOHOL] (phase B).

Phase B was slowly added to phase A.

| | INGREDIENT (INCI Nomenclature) | % IN WEIGHT |
|---|---|---|
| A | DIETHYLHEXYL SODIUM SULFOSUCCINATE/ ISOSTEARIC ACID (15/85) | 89.50 |
| B | CAFFEINE | 0.50 |
| B | WATER (AQUA) | 5.00 |
| B | ALCOHOL | 5.00 |

### Example 10-f: Preparation of lipid nanoparticle capsules containing microemulsified caffeine.

In a suitable vessel water, Structure XL [INCI: HYDROXYPROPYL STARCH PHOSPHATE] and Amigel [INCI: SCLEROTIUM GUM] were mixed together. Stir until a homogeneous solution is observed. Next, hyaluronic acid [INCI: SODIUM HYALURONATE] was added (ingredients A).

In another vessel the caffeine microemulsion (example *10-e*), Lanette O [INCI: CETEARYL ALCOHOL], Emulgade SE DF [INCI: CETEARETH-12] and Arlamol HD [INCI: ISOHEXADECANE] were mixed together (ingredients B). The mixture was heated until all the ingredients had merged.

Mixture B was added to mixture A under continual stirring, maintaining the two mixtures at 70-80°C until a good emulsion was obtained. Stirring was continued for 15 minutes.

Finally, the mixture was homogenized under pressure in a microfluidizer for 3 cycles with an entrance pressure of 80 bar and an exit pressure of 15000 psi. Next, the homogenized mixture was allowed to cool to room temperature whilst being stirred with a turbine.

| | INGREDIENT (INCI Nomenclature) | % IN WEIGHT |
|---|---|---|
| A | WATER (AQUA) | QSP100 |
| A | HYDROXYPROPYL STARCH PHOSPHATE | 1.00 |
| A | SCLEROTIUM GUM | 0.50 |
| A | SODIUM HYALURONATE | 0.01 |
| B | DIETHYLHEXYL SODIUM SULFOSUCCINATE/ ISOSTEARIC ACID (15/85), CAFFEINE, WATER (AQUA), ALCOHOL | 20.00 |
| B | CETEARYL ALCOHOL | 2.00 |
| B | CETEARETH-12 | 3.50 |
| B | ISOHEXADECANE | 1.00 |

The average size of the capsules in suspension obtained determined by Dynamic Laser Light Scattering was 99.9 nm.

### Example 10-g: Preparation of lipid nanoparticle capsules containing microemulsified caffeine.

In a suitable vessel Centrolex F [INCI: LECITHIN] was added little by little to water until the correct dispersion was achieved (ingredient A1).

In another vessel, hyaluronic acid [INCI: SODIUM HYALURONATE] was dissolved in water (ingredients A2).

Next, solution A2 was added to A1 under continual stirring (mixture A). Mixture A was heated in a microwave to 80-85°C.

In another vessel, Lipochroman-6 [INCI: DIMETHYLMETHOXY CHROMANOL], caffeine microemulsion (example *10-e*), Cutina CP [INCI: CETYL PALMITATE], Cutina CBS [INCI: COCOGLYCERIDES], Inutec SP1 [INCI: INULIN LAURYL CARBAMATE] and Dermofeel PS [INCI: POLYGLYCERYL-3 STEARATE] were mixed together (ingredients B). The mixture was heated until all the ingredients had merged.

Next, mixture B was added to mixture A under continual stirring until a good emulsion was obtained.

The hot mixture obtained (T>75°C) was homogenized under pressure in a microfluidizer for 3 cycles with an entrance pressure of 80 bar and an exit pressure of 15000 psi.

Once the mixture had been microfluidified, a suspension in water of Quat Soy LDMA 25 [INCI: LAURYLDIMONIUM HYDROXYPROPYL HYDROLYZED SOY PROTEIN] was added drop by drop under stirring (ingredients C).

The mixture was allowed to cool to room temperature under stirring. The pH was adjusted to between 5-6 with NaOH.

Next, Structure XL [INCI: HYDROXYPROPYL STARCH PHOSPHATE] and Amigel [INCI: SCLEROTIUM GUM] were added (ingredients D), and the mixture was stirred until a homogeneous solution was observed.

Finally, it was allowed to cool whilst being stirred with a turbine.

| | INGREDIENT (INCI Nomenclature) | % IN WEIGHT |
|---|---|---|
| A1 | WATER (AQUA) | QSP100 |
| A1 | LECITHIN | 5.00 |
| A2 | WATER (AQUA) | 10.00 |
| A2 | SODIUM HYALURONATE | 0.01 |
| B | DIETHYLHEXYL SODIUM SULFOSUCCINATE/ ISOSTEARIC ACID (15/85), CAFFEINE, WATER (AQUA), ALCOHOL | 20.00 |
| B | DIMETHYLMETHOXY CHROMANO | 0.015 |
| B | CETYL PALMITATE | 1.00 |
| B | COCOGLYCERIDES | 1.00 |
| B | INULIN LAURYL CARBAMATE | 1.00 |
| B | POLYGLYCERYL-3 STEARATE | 1.00 |
| C | WATER (AQUA) | 2.00 |
| C | LAURYLDIMONIUM HYDROXYPROPYL HYDROLYZED SOY PROTEIN | 0.02 |
| D | HYDROXYPROPYL STARCH PHOSPHATE | 1.00 |
| D | SCLEROTIUM GUM | 0.50 |

The average size of the capsules in suspension obtained determined by Dynamic Laser Light Scattering was 137 nm.

### Percutaneous absorption test

In the percutaneous permeation studies, the formulation studied was applied to a sample of skin placed in a Franz diffusion cell. The exposure of the skin to the formulation was maintained for 24 hours. After the 24 hours, the receptor fluid was collected, the skin was washed to eliminate the excess preparation and the different layers of the skin were subsequently evaluated by HPLC.

The following table shows the results obtained:

| **SAMPLE** | **Quantity percutaneously absorped (% dosis applied)** |
|---|---|
| *10-a* | 2.37 |
| *10-b* | 0.99 |
| *10-c* | 4.31 |
| *10-d* | 1.47 |
| *10-e* | 6.42 |
| *10-g* | 10.47 |
| *10-f* | 18.10 |

It could be clearly observed that in the case of the lipid nanoparticle capsules, independently from the type of formulation (*10-a, 10-b*), there was an important increase in cutaneous permeation with regards to the other delivery systems assayed.

This result allows the conclusion to be made that the lipid nanoparticles capsules constitute a very suitable delivery system for incorporation in cosmetic and/or pharmaceutical compositions applied to the skin.

## Claims

1. Delivery system which comprises lipid nanoparticles selected from the group formed by solid lipid nanoparticles and nanostructured lipid carriers, containing at least one active ingredient and which are polymerically coated with a coacervate.

2. Delivery system according to claim 1, wherein the lipids in the lipid nanoparticles can be solid lipids or a mixture of liquid lipids and solid lipids at room temperature.

3. Delivery system according to claim 2, wherein the liquid or semi-liquid lipids are selected from the group formed by vegetable oils, soybean oil, sunflower oil, corn oil, olive oil, palm oil, cotton seed oil, colza oil, peanut oil, coconut oil, castor oil, linseed oil, borage oil, evening primrose oil, marine oils, fish oils, algae oils, oils derived from petroleum, mineral oil, liquid paraffin, vaseline, short-chain fatty alcohols, medium-chain aliphatic branched fatty alcohols, fatty acid esters with short-chain alcohols, isopropyl myristate, isopropyl palmitate, isopropyl stearate, dibutyl adipate, medium-chain triglycerides, capric and caprylic acid triglycerides, C₁₂-C₁₆ octanoates; fatty alcohol ethers, dioctyl ether and/or mixtures thereof.

4. Delivery system according to claim 2, wherein the solid lipids are selected from the group formed by solid triglycerides, trilaurin, tricaprylin, tripalmitin, tristearin, glyceryl trilaurate, glyceryl trimyristate or trimyristin, glyceryl tripalmitate, glyceryl tristearate, glyceryl behenate or tribehenin, solid diglycerides, dipalmitin, distearin, solid monoglycerides, glyceryl monostearate, glyceryl palmitostearate, glyceryl stearate citrate, long-chain aliphatic alcohols, cetyl alcohol, stearic alcohol, medium and long-chain fatty acids (C₁₀-C₂₂), stearic acid, palmitic acid, behenic acid and capric acid, fatty alcohol esters with long and medium-chain fatty acids with polyols (C₁₀-C₂₂), fatty alcohol esters of long-chain fatty acids, cetyl palmitate, cetearyl olivate, hydroxyoctacosanyl hydroxystearate, sterols, cholesterol, cholesterol esters, cholesteryl hemisuccinate, cholesteryl butyrate, cholesteryl palmitate, fatty amines, stearyl amine, waxes, beeswax, shea butter, cocoa butter, carnauba wax, ozokerite wax, paraffin wax, ceramides, hydrogenated vegetable oils, hydrogenated castor oil, quaternary ammonium derivatives, behenyl trimethyl ammonium chloride, and/or mixtures thereof.

5. Delivery system according to claim 1, wherein the polymer in the coating of this delivery system is selected from the group formed by proteins, polysaccharides, polyesters, polyacrylates, polycyanoacrylates and/or mixtures thereof.

6. Delivery system according to claim 5, wherein the polymer in the coating of this delivery system is selected from the group formed by gelatin, albumin, soy protein, pea protein, broad bean protein, potato protein, wheat protein, whey protein, β-lactoglobulin, caseinates, wheat starch, corn starch, zein, alginates, carrageenans, pectins, arabinogalactans, gum arabic, xanthan gum, mesquite gum, tragacanth gum, galactomannans, guar gum, carob seed gum, chitosan, agar, poly(L-lysine), dextran sulfate sodium, carboxymethyl galactomannan, carboxymethyl cellulose, methyl cellulose, ethyl cellulose, hydroxypropyl methyl cellulose, cellulose nitrate, cellulose acetate butyrate, cellulose acetate phthalate, hydroxypropyl methylcellulose phthalate, hydroxypropyl methylcellulose acetate succinate, polyvinyl acetate phthalate, poly(ε-caprolactone), poly(*p*-dioxanone), poly(δ-valerolactone), poly(β-hydroxybutyrate), poly(β-hydroxybutyrate) and β-hydroxyvalerate copolymers, poly(β-hydroxypropionate), methylacrylic acid copolymers, dimethylaminoethyl methacrylate copolymers, trimethylammonium ethyl methacrylate copolymers, lactic and glycolic acid polymers and copolymers, lactic and glycolic acid polymers and copolymers and polyethylene glycol and mixtures thereof.

7. Delivery system according to claim 1, wherein the polymer in the polymeric coating of this delivery system is a cationic polymer.

8. Delivery system according to claim 7, wherein the cationic polymer is selected from the group formed by cationic cellulose derivatives, quaternized hydroxyethylcellulose, cationic starches, diallyl ammonium and acrylamide salt copolymers, quaternized vinylpyrrolidone/vinylimidazole polymers, condensation products of polyglycols and amines, polyquaternium polymers and copolymers, polymers called polyquaternium-6, polyquaternium-7, polyquaternium-16, polyquaternium-10 Merquats, polyquaternium-4 copolymers, dicocoylethylhydroxyethylammonium, grafting copolymers with a cellulose skeleton and quaternary ammonium groups, quaternized collagen polypeptides, laurdimonium hydroxypropyl hydrolyzed collagen, quaternized wheat polypeptides, polyethylenimine, cationic silicone polymers, amidomethicone or silicone quaternium-22, adipic acid and dimethylamino hydroxypropyl diethylenetriamine copolymers, acrylic acid copolymers with dimethyldiallylammonium chloride, cationic chitin derivatives, condensation products of cationic dihalogen alkylene, condensation products of dibromobutane with bisdialkylamines, bis-dimethylamino-1,3-propane, derivatives of cationic guar gum, guar-hydroxypropyltrimonium, quaternary ammonium salt polymers, quaternized polysaccharide polymers of natural derivatives such as azarose, cationic gelatin proteins, cationic gum arabic proteins, cationic polyamide polymers, cationic polycyanoacrylate polymers, cationic polylactide polymers, cationic polyglycolides polymers, cationic polyaniline polymers, cationic polypyrrole polymers, cationic polyvinylpyrrolidone polymers, cationic polymers of amino silicone polymers and copolymers, cationic polystyrene polymers, cationic polyvinyl alcohol polymers, cationic polystyrene and maleic acid anhydride copolymers, cationic methyl vinyl ether polymers, cationic epoxy resin polymers, cationic polymers of styrene and methyl methacrylate copolymers, dimethylamino methacrylate, cationic polyacrylates and polymethacrylates, polyamine derivatives optionally substituted by derivative polyethylene glycol members, polyamino acids under pH conditions wherein they are cationic, polyethyleneimine, quaternized derivatives of polyvinylpyrrolidone and hydrophilic urethane polymers, as well as any mixture of the aforementioned cationic groups.

9. Delivery system according to claim 1, wherein the active ingredient is selected from the group formed by cosmetic, pharmaceutical and/or alimentary active ingredients and/or adjuvants.

10. Delivery system according to claim 9, wherein the cosmetic and/or alimentary active ingredients and/or adjuvants are selected from the group formed by surfactants, humectants or substances which retain moisture, moisturizers or emollients, agents stimulating healing, coadjuvant healing agents, agents stimulating re-epithelialization, coadjuvant re-epithelialization agents, agents which synthesize dermal or epidermal macromolecules, firming and/or redensifying and/or restructuring agents, cytokine growth factors, agents which act on capillary circulation and/or microcirculation, anti-glycation agents, free radical scavengers and/or anti-atmospheric pollution agents, reactive carbonyl species scavengers, 5α-reductase-inhibiting agents, lysyl- and/or prolyl hydroxylase inhibiting agents, defensin synthesis-stimulating agents, bactericidal agents and/or bacteriostatic agents and/or antimicrobial agents and/or germicidal agents and/or fungicidal agents and/or fungistatic agents and/or germ-inhibiting agents, anti-viral agents, antiparasitic agents, antihistaminic agents, NO-synthase inhibiting agents, desquamation agents or keratolytic agents and/or exfoliating agents, comedolytic agents, anti-psoriasis agents, anti-dandruff agents, anti-inflammatory agents and/or analgesics, anesthetic agents, anti-wrinkle and/or anti-aging agents, cosmetic and/or absorbent and/or body odor masking deodorants, antiperspirant agents, perfuming substances and/or perfumed oils and/or isolated aromatic compounds, anti-oxidizing agents, agents inhibiting vascular permeability, hydrolytic epidermal enzymes, whitening or skin depigmenting agents, agents inhibiting sweat-degrading enzymes, agents capable of filtering UV rays, agents which stimulate or regulate keratinocyte differentiation, anti-itching agents, agents which stimulate or inhibit the synthesis of melanin, propigmenting agents, self-tanning agents, agents stimulating the proliferation of melanocytes, liquid propellants, vitamins, amino acids, proteins, biopolymers, gelling polymers, skin relaxant agents, agents capable of reducing or treating bags under eyes, agents for the treatment and/or care of sensitive skin, astringent agents, agents regulating sebum production, anti-stretch mark agents, lipolytic agents or agents stimulating lipolysis, venotonic agents, anti-cellulite agents, calming agents, agents acting on cell metabolism, agents to improve dermal-epidermal junction, agents inducing hair growth or hair-loss retardants, body hair growth inhibiting or retardant agents, heat shock protein synthesis stimulating agents, muscle relaxants, muscle contraction inhibitory agents, agents inhibiting the aggregation of acetylcholine receptors, anticholinergic agents, elastase inhibitory agents, matrix metalloproteinase inhibitory agents, chelating agents, vegetable extracts, essential oils, marine extracts, mineral salts, cell extracts, emulsifying agents, agents stimulating the synthesis of lipids and components of the stratum corneum, agents obtained from a bio-fermentation process and/or mixtures thereof.

11. Delivery system according to claim 9, wherein the pharmaceutical active ingredients and/or adjuvants are selected from the group formed by antiacids, agents against peptic ulcers and gastroesophageal reflux disease, antispasmodics, analgesics, anticholinergic drugs, propulsive drugs, antiemetics, antinausea drugs, agents for biliary therapy, agents for hepatic therapy, lipotropics, laxatives, antidiarrhetics, intestinal adsorbents, antipropulsives, anti-inflammatory drugs, active ingredients against obesity, digestive agents, enzymes, hypoglycemic drugs, insulin, vitamins, proteins, minerals, anabolic steroids, antithrombotic agents, antifibrinolytics, haemostatic agents, antiarrhythmic agents, cardiac stimulants, cardiac glycosides, vasodilators, antiadrenergic agents, antihypertensive drugs, diuretics, potassium-saving agents, antihemorrhoidals, antivaricose therapy agents, capillary stabilizing agents, agents which act on the renin-angiotensin system, beta-blockers, selective calcium-channel blockers, non-selective calcium-channel blockers, ACE inhibitors, angiotensin II inhibitors, modifying agents of lipids, antifungals, healing agents, antipruritics, antihistamines, anesthetics, antipsoriatics, chemotherapy drugs, corticosteroids, antiseptics, disinfectants, anti-acne agents, products for gynecological use, oxytocics, anticonceptives, androgen, estrogen, progestagen, gonadotropins, ovulation stimulants, antiandrogens, products for urological use, antispasmodics, drugs used in benign prostatic hypertrophy, hormones, hormone antagonists, antibiotics, tetracyclines, anphenicols, beta-lactam antibacterials, penicillin, sulfonamides, trimethoprim, macrolides, lincosamides, streptogramins, antibacterial aminoglycosides, antibacterial quinolones, antivirals, immune serum, immunoglobulins, antineoplastic agents, immunomodulatory agents, alkylation agents, antimetabolites, plant alkaloids, cytotoxic antibiotics, immunosuppressive agents, drugs for disorders of the musculoskeletal system, antirheumatics, muscle relaxant agents, agents which affect bone structure and mineralization, drugs which act on the nervous system, general anesthetics, local anesthetics, opioids, antimigraine agents, anticonvulsants, anticholinergic agents, dopaminergics, antipsychotics, anxiolytics, hypnotics, sedatives, antidepressants, psychostimulants, anti-dementia drugs, parasympathomimetics, drugs used in addictive disorders, anti-vertigo agents, antiparasitic agents, insecticides, insect repellants, nasal decongestants, mucolytic agents, cough suppressants, ophthalmic active ingredients, otological active ingredients, antiglaucoma drugs, miotics, mydriatics, cycloplegics and/or mixtures thereof.

12. Cosmetic, pharmaceutical and/or alimentary composition which comprises the delivery system according to any of claims 1 to 11.

13. Composition according to claim 12, wherein this composition is presented in a formulation selected from the group formed by creams, multiple emulsions, anhydrous compositions, aqueous dispersions, oils, milks, balsams, foams, lotions, gels, cream gels, hydroalcoholic solutions, hydroglycolic solutions, hydrogels, liniments, sera, soaps, shampoos, conditioners, serums, ointments, mousses, pomades, powders, bars, pencils, vaporizers, aerosols, capsules, gelatin capsules, soft capsules, hard capsules, tablets, sugar coated tablets, granules, chewing gum, solutions, suspensions, emulsions, syrups, polysaccharide films, jellies and gelatin.

14. Use of the cosmetic, pharmaceutical and/or alimentary composition according to claim 12 for the treatment and/or care of the skin, scalp, hair and nails.

15. Use of the cosmetic and/or pharmaceutical composition according to claim 12 for the treatment of textile materials.
